## Europäisches Patentamt
## European Patent Office
## Office européen des brevets

(19) 

(11) Numéro de publication: **0 114 777**
**B1**

(12) **FASCICULE DE BREVET EUROPEEN**

(45) Date de publication du fascicule du brevet: **01.08.90**

(21) Numéro de dépôt: **84400126.3**

(22) Date de dépôt: **20.01.84**

(51) Int. Cl.⁵: **C 12 N 15/00**, C 12 P 21/02, C 12 N 1/20, A 61 K 37/64 // C12R1/19

(54) **Nouveaux vecteurs d'expression et leur application à la préparation d'une protéine ayant l'activité de l'antitrypsine-alpha 1 humaine.**

(30) Priorité: **21.01.83 FR 8300909**
**12.07.83 FR 8311594**

(43) Date de publication de la demande:
**01.08.84 Bulletin 84/31**

(45) Mention de la délivrance du brevet:
**01.08.90 Bulletin 90/31**

(84) Etats contractants désignés:
**AT BE CH DE FR GB IT LI LU NL SE**

(56) Documents cités:
EP-A-0 038 182
EP-A-0 041 767
EP-A-0 094 887
EP-A-0 099 084
FR-A-1 431 678

GENE, vol. 13, 1981, pages 289-298, Elsevier/North-Holland,Biomedical Press, NL; A. HONINGMAN et al: "Plasmid vectors for positive selection of DNA inserts controlled by the lambda pL promoter repressor and antitermination function"

(73) Titulaire: **TRANSGENE S.A.**
**19 Rue Henri Regnault**
**F-92411 Courbevoie Cédex (FR)**

(72) Inventeur: **Courtney, Michael**
**18 rue du Ballon**
**F-67100 Strasbourg (FR)**
Inventeur: **Buchwalder, Anne**
**13 rue du Bain Aux Plantes**
**F-67000 Strasbourg (FR)**
Inventeur: **Tessier, Luc-Henri**
**20, rue du Vieux Marché aux Grains**
**F-67000 Strasbourg (FR)**
Inventeur: **Jaye, Michael**
**6 rue des Dentelles**
**F-67000 Strasbourg (FR)**
Inventeur: **Lathe, Richard**
**8 Quai des Bateliers**
**F-67000 Strasbourg (FR)**
Inventeur: **Tolstoshev, Paul**
**6 rue Bartholdi**
**F-67200 Mittelhausbergen (FR)**
Inventeur: **Lecocq, Jean-Pierre**
**6 rue du Champ du feu**
**F-67116 Reichstett (FR)**

(74) Mandataire: **Warcoin, Jacques et al**
**Cabinet Régimbeau 26, avenue Kléber**
**F-75116 Paris (FR)**

Courier Press, Leamington Spa, England.

**EP  0 114 777  B1**

(56) References cited:
GENE, vol. 15, 1984, pages 81-93,
Elsevier/North-Holland, Biomedical Press, NL;
E. REMAUT et al.: "Plasmid vectors for higheffeciency expression controlled by the pL
promoter of coliphage lambda"

PROC. NATL. ACAD. SCI., vol. 78, no. 11,
novembre 1981, pages 6826-6830, US; K.
KURACHI et al.: "Cloning and sequence of cDNA
coding for alpha1-antitrypsin"

**Description**

La présente invention concerne de nouveaux vecteurs de clonage et d'expression, des bactéries transformées à l'aide de ces vecteurs et leurs applications pour la préparation d'une protéine ayant l'activité de l'antitrypsine-$\alpha_1$ humaine.

L'antitrypsine-$\alpha_1$ est une glycoprotéine sérique représentant 90% des globulines $\alpha_1$ dans le sérum humain. Elle est présente à concentration de 130 à 150 mg/dl dans le sérum normal. Il s'agit d'une chaîne polypeptidique simple ayant un poids moléculaire de 47—52 000 daltons, 10 à 15% du poids étant des chaînes latérales d'hydrates de carbone. De nombreuses variantes humaines de l'antitrypsine-$\alpha_1$ sont connues et la plupart de ces polymorphismes semblent résulter chaque fois d'un seul substituant aminoacide. L'antitrypsine-$\alpha_1$ est synthétisée essentiellement dans le foie.

La fonction de l'antitrypsine-$\alpha_1$ est celle d'une antiprotéase; elle se combine avec une grande variété de protéases et les rend inactives, notamment la trypsine, la thrombine, la chimotrypsine et la plasmine.

Toutefois, sa fonction essentielle est d'inhiber l'élastase neutrophile, une protéase à large spectre capable de dégrader la plupart des protéines de structure, en particulier les composants du tissu conjonctif.

Bien qu'il s'agisse d'une protéine sérique circulante, l'antitrypsine-$\alpha_1$ présente une importance essentielle comme inhibiteur de l'élastase extravasculaire. Ceci est dû essentiellement à sa petite taille qui lui permet, à la différence de l'antiélastase sérique plus grosse, la macroglubuline-$\alpha_2$, de diffuser à travers la plupart des tissus.

Ainsi, approximativement 44% de l'antitrypsine-$\alpha_1$ est extravasculaire et les organes les plus importants protégés par l'antitrypsine-$\alpha_1$ sont les poumons.

Une déficience en antitrypsine-$\alpha_1$ peut provoquer un déséquilibre protéase/antiprotéase et, pour les raisons indiquées précédemment, les conséquences cliniques les plus significatives se produisent au niveau des poumons. Dans ces organes, en effet, un excès d'activité élastasique conduit avec une fréquence élevée à des maladies endommageant le poumon tel que l'emphysème.

Le cas le plus connu de déficience en antitrypsine-$\alpha_1$ est dû à un désordre génétique au cours duquel certains phénotypes antitrypsine-$\alpha_1$ variants sont associés à une déficience marquée en antitrypsine-$\alpha_1$ sérique. Le variant le mieux étudié, le type Z, est une substitution d'aminoacide (la lysine remplace l'acide glutamique en position 53 à partir de l'extrémité C terminale de la protéine). Ce changement affecte l'importance de la glycosilation de la molécule d'antitrypsine-$\alpha_1$, ce qui conduit à un défaut de sécrétion de la molécule, une accumulation dans les hépatocytes et un taux sérique en antitrypsine-$\alpha_1$ qui représente de 10 à 15% de celui des contrôles. Cette déficience est associée à un développement de l'emphysème chez les individus âgés de 40 à 50 ans et moins fréquemment avec des troubles du foie. L'allèle Z a une fréquence située entre 1/3 000 et 1/4 000 dans la population caucasoïde des Etats-Unis d'Amérique, ainsi qu'en Europe. Ceci correspond approximativement à 50 000 homozygotes ZZ aux Etats-Unis d'Amérique. De plus, environ une personne sur 800 aux E.U.A. présente le phénotype SZ qui est associé à un taux d'antitrypsine-$\alpha_1$ dans le sérum qui est seulement de 35% de la normale. Ce taux est probablement le seuil correspondant au développement de destruction an niveau des poumons par un déséquilibre protéase/antiprotéase et le nombre de personnes affectées aux E.U.A. est de l'ordre de 250 000. Les fréquences européennes sont approximativement du même ordre (références 1 à 3).

Il est clair que tout facteur qui réduit le taux de l'antitrypsine-$\alpha_1$ fonctionnelle conduit à un déséquilibre protéase/antiprotéase et prédispose par là même à des atteintes pulmonaires. Il est maintenant clairement établi qui de tels désordres apparaissent chez les fumeurs de cigarettes, la fumée de cigarettes constituant l'un des facteurs majeurs qui sont mis en cause dans l'emphysème non-héréditaire. Il a été démontré à la fois que les composants de la fumée de cigarettes peuvent inhiber fonctionnellement l'antitrypsine-$\alpha_1$ en oxydant un résidu méthionine près du site d'inhibition de l'élastase (référence 4) et que l'activité fonctionnelle de l'antitrypsine-$\alpha_1$ est au moins réduite de deux fois dans les poumons des fumeurs (référence 5).

Il est intéressant de noter que les fumeurs ne présentent pas de baisse de l'antitrypsine-$\alpha_1$ sérique, mais que cette déficience est localisée dans les poumons et est due à l'action directe de la fumée de cigarette. Il est très probable que la fumée de cigarette peut également induire un taux élevé d'élastase neutrophile dans les poumons, en accroissant ainsi la charge protéolytique sur les poumons qui sont moins protégés compte tenu de l'inactivation de l'antitrypsine-$\alpha_1$.

Divers autres étas pathologiques peuvent être associés avec la reduction du taux sérique de l'antitrypsine-$\alpha_1$, les plus significatifs sont, notamment, les syndromes de difficultés respiratoires aiguës chez les nouveau-nés et les adultes, les rhumatismes, l'arthrite, l'asthme stéroïdodépendant et probablement les fibroses cystiques (référence 1).

Il est clair que tout déséquilibre protéase/antiprotéase, qui peut avoir une manifestation sur le plan clinique (référence 6), doit pouvoir être traité par une thérapie de remplacement.

Les premières approches de cette techniques ont consisté à effectuer des injections intraveineuses d'antitrypsine-$\alpha_1$ humaine préparée à partir de sérum normal aux individus présentant une déficience génétique (homozygotes ZZ). Le résultat de ces études préliminaires montre que le taux sérique d'antitrypsine-$\alpha_1$ peut être maintenu à une valeur supérieure à 70 mg/dl, c'est-à-dire un niveau suffisant pour assurer une protection anti-élastase efficace des poumons. Il est évident que toute déficience en antitrypsine-$\alpha_1$ peut être approchée thérapeutiquement de cette façon.

Pour un tel type de thérapie, il est nécessaire de disposer de grandes quantités d'ATαI humaine très pure. Mais l'extraction de l'ATαI à partir du sérum se heurte aux problèmes de purification des protéines extraites du milieu naturel et également aux problèmes liés à l'utilisation de sérum comme matière de base qui sont essentiellement des problèmes de contaminants.

Ces différents problèmes peuvent être résolus en utilisant un produit obtenu par fermentation bactérienne — comme cela sera démontré ci-après —.

En effet, il est connu de purifier des polypeptides tels qu'enzymes, antigènes et hormones, à partir de culture de bactéries incorporant un vecteur d'expression, tel qu'un plasmide, dans lequel se trouve cloné le gène codant pour lesdites polypeptides.

Afin de préciser certains éléments de terminologie qui ser ont utilisés dans le cadre de la description, il convient de rappeler brièvement les éléments principaux régissant l'expression d'un gène.

Pour que le gène cloné puisse "s'exprimer", c'est-à-dire que le produit désiré codé par ledit gène soit synthétisé par la bactérie, il convient que le vecteur utilisé présente certaines caractéristiques qui sont liées à l'expression des gènes.

L'expression d'un gène cloné comporte deux grandes étapes:
— tout d'abord la transcription de l'information codée par l'ADN du vecteur en ARN messager (mARN);
— puis la traduction de cet mARN en polypeptides au niveau des ribosomes.

L'étape de transcription commence par la fixation d'une enzyme, l'ARN polymérase qui assure la "lecture" de l'information, sur une partie de l'ADN du vecteur appelée "promoteur" qui initie la transcription.

Cette fixation est soumise à différents types de régulation, en particulier la transcription par l'ARN polymérase peut être réprimée par la fixation d'une protéine dénommée "répresseur" qui se fixe sur une sequence dénommée "opérateur", au voisinage du promoteur (contrôle négatif).

C'est seulement lorsque le répresseur est désactivé que l'ARN polymérase peut commencer la transcription.

La désactivation du répresseur ou induction est effectuée soit par action d'une substance agissant sur le répresseur, soit par d'autres moyens tels que la chaleur.

L'ADN une fois transcrit en mARN par l'intermédiaire de l'ARN polymérase, le mARN va être traduit au niveau des ribosomes en une chaîne polypeptide.

Pour que cette traduction puisse s'effectuer, il est nécessaire que le mARN comprenne une région de fixation des ribosomes et le site d'initiation de la traduction: L'ADN du vecteur doit présenter les séquences correspondants appelées ci-après "région d'initiation de la traduction".

Si les éléments mentionnés précédemment sont nécessaires pour l'expression du gène cloné, le vecteur d'expression possède également une autre propriété intéressante. En effet, il est capable de se répliquer et de se multiplier dans certaines cellules bactériennes dites "cellules hôtes" et, par là même, d'augmenter le nombre de "copies" du gène cloné. Ceci conduit, dans la plupart des cas où le gène est exprimé, à une production accrue de la protéine.

Enfin, le vecteur d'expression, en particulier lorsqu'il s'agit d'un plasmide, présente en général des gènes codant pour la résistance à différents antibiotiques, ce type de résistance est utilisé comme "marqueur" permettant de sélectionner les souches bactériennes transformées.

D'autres éléments intéressants der vecteurs d'expression seront explicités ci-après dans le cours de la description.

La présente invention concerne un nouveau vecteur de clonage et d'expression permettant une production améliorée de protéines et/ou de peptides dans une souche de bactérie gram négative et en particulier *Escherichia coli*.

Plus particulièrement, il s'agit d'un vecteur de clonage et d'expression d'un gène déterminé dans une bactérie gram négative, caractérisé en ce qu'il comporte:
— l'origine de réplication d'un plasmide bactérien,
— un promoteur du bactériophage λ: $P_L$, $P_R$ ou $P'_R$;
— une région codant pour l'initiation de la traduction,
— une région de clonage comportant des sites de restriction uniques.

Comme cela a été indiqué précédemment, la présence d'une origine de réplication pour un plasmide est essentielle pour permettre la réplication du vecteur dans les cellules bactériennes correspondantes, en particulier dans le cas de E. coli on utilisera, de préférence, l'origine de réplication du plasmide pBR322. Le plasmide pBR322 présente, en effet, l'avantage de donner un grand nombre de copies et ainsi d'augmenter la quantité de plasmides produisant la protéine désirée.

Il est, bien entendu, possible d'utiliser d'autres origines de réplication, en particulier celle d'un plasmide codant pour les facteurs de résistances quelconques ou d'un épisome colicinogénique (c'est-à-dire conférant à une bactérie la faculté de synthétiser une colicine).

Toutefois, il convient de choisir des plasmides dont la réplication n'est pas contrôlée de façon trop sévère et qui ne présente pas de site de restriction indésirable, en dehors de la région de clonage.

Parmi les promoteurs du bactériophage λ, on utilisera, de préférence, le promoteur principal de gauche noté $\lambda P_L$ est un promoteur puissant responsable de la transcription précoce de λ. Des publications antérieures font déjà état de l'expression à un haut niveau des gènes clonés sous le contrôle de $P_L$ (références 8 et 9).

EP 0 114 777 B1

Il est également possible d'utiliser d'autres promoteurs du bactériophage λ, notamment le promoteur de droite, P'R, ou le second promoteur de droite, P'R.

Bien qu'il soit possible d'utiliser des séquences d'initiation de la traduction très variées, on préfère utiliser celle de la protéine cII du bactériophage λ qui sera nommée ci-après λcIIrbs.

Si l'utilisation de la région λcIIrbs est plus particulièrement intéressante, pour des raisons qui seront explicitées ci-après, il est néanmoins possible d'utiliser d'autres séquences d'initiation de la traduction, notamment celle du gène λE qui est limitée par des sites de restriction intéressants, mais également celles de:

R17 COAT    CCTCAACCGGGGTTTGAAGCATGGCTTCTAACTTT

QB COAT    GAAACTTTGGGTCAATTTGATCATGCAAAATTAGAGACTAGACTGA

F2 COAT    CCTCAACCGAGGTTTGAAGCATGGCTTCCAACTTACTCAG

MS2 COAT    GAGCCCTCAACCGGAGTTTGAAGCATGGCTTCTAACTTTACTCAGTT

PHIX D    TTCAACCACTAATAGGTAAGAAATCATGAGTCAAGTTTACTGAACAATC

Sau96I                                                                Narl
*PHIX F    TTCGGCCCCTTACTTGAGGATAAATTATGTCTAATATTCAAACTGGCGCCG

G4 D    TGACACAAACCACAAAGGAAACTGAAATGTCTAAATCAAACGAATCTGCTG

G4 F    GTCCCACTCTATTTAAGGATACAAAAATGTCTAACGTTCAAACATCTGCGG

L 11    TTATTACCCAACTTGAGGAATTTATAATGGCTAAGAAAGTACAAAGCCTATG

L 1    TGGGCCTGGTAGTGGAGGACTAAGAAATGGCTAAACTGACCAAGCGCATGCG

L7    CTGGCAAACATCCAGGAGCAAAGCTAATGGCTTTAAATCTTCAAGACAAACAA

L 12    TATTCTGATATTCAGGAACAATTTAAATGTCTATCACTAAAGATCAAATCATT

RPO B    GACTTGTCAGCGAGCTGAGGAACCCTATGGTTTACTCCTATACCGAGAAAAAA

SPC    AGTAGTTGACATTAGCGGAGCCTAAAATGATCCAAGAACAGACTATGCTGAAC

STR    CAAAAGCTAAAACCAGGAGCTATTTAATGGCAACAGTTAACCAGCTGGTACGC

*CII    ATTGTTATCTAAGGAAATACTTACATATGGTTCGTGCAAACAAACGCAACGAG

TUF B    CGATTTACCGTGTCTTAGAGGGACAATCGATGTGTAAAGAAAAGTTTGAACGTACA

OMP A    ATACAGTAACTCACAGGGGCTGGATTGATTATGTACACTTCAGGCTATGCACAT

LAM—D    TGAACACACCAGTGTAAGGGATGTTTATGACGAGCAAAGAAACCTTTACCCATT

LAM—E    TGTGCGGCTTTTTTTACGGGATTTTTTTATGTCGATGTACACAACCGCCCAACTGCTG

Dans certains cas on utilisera une séquence d'initiation synthétique en particulier:

ATCGA TAA CAC AGGAA CAGATCT ATG
         ***    .............

Cette séquence d'initiation comporte une séquence Shine/Dalga o (S/D) AGGAA et un codon de terminaison de la traduction TAA qui stoppe la lecture de N dont on verra ci-après l'utilité.

La region codant pour l'initiation de la traduction λcIIrbs est plus particulièrement intéressante car:

1°) il a été démontré que la protéine cII est synthétisée en grande quantité sous le contrôle du promoteur PL;

2°) La région en cause est limitée par des sites de restriction qui permettent un isolement facile de ladite région;

3°) une enzyme de restriction (NdeI) recouvre le codon d'initiation de la traduction.

Dans ces conditions, l'insertion des gènes étrangers sur ce site avec reconstruction du codon

5

d'initiation ATG permet l'expression de protéines non-fusionnées, c'est-à-dire ne contenant pas d'aminoacide étranger à la protéine que l'on devra préparer à l'exception de la méthionine initiale.

Par contre, le clonage d'un gène étranger dans l'un des sites de restriction uniques en aval de λcIIrbs conduit à l'expression d'une protéine fusionnée comme cela sera décrit ci-dessous.

Il est intéressant de pouvoir obtenir selon les besoins des protéines fusionnées ou non-fusionnées.

Une protéine fusionnée comporte en général, outre la protéine intéressante, une partie provenant de l'expression de gènes du vecteur ou de certains éléments de jonction des différentes éléments du vecteur.

Ceci nécessite donc par la suite un clivage de la protéine exprimée pour libérer la partie protéique intéressante. Toutefois, dans certains cas il est possible de faire excréter la protéine fusionnée.

Au contraire, dans le cas de protéine non fusionnée, il n'est pas nécessaire de prévoir un clivage mais la protéine n'est, en général, pas excrétée et doit donc être isolée d'un lysat bactérien.

Enfin, dans certains cas, une protéine non fusionnée ne sera pas stable dans la cellule alors que la protéine fusionnée le sera.

Il faut donc, dans chaque cas, étudier les avantages et les inconvénients, et il est important de disposer d'un vecteur polyvalent pouvant permettre l'expression des deux types de protéines.

La zone de clonage comportant des sites de restriction uniques permet l'insertion de gènes étrangers pour la production de protéines fusionnées (ou non fusionnées). Ces sites de restriction uniques sont situés en aval du promoteur $P_L$ et de la région d'initiation de traduction de cII. Parmi les sites d'insertion uniques qui figurent de préférence dans cette zone, on peut citer EcoRI, SalI, AccI, BamHI, HindIII, HindII et PstI correspondant à des enzymes de restriction couramment utilisés, L'insertion des gènes codant pour une protéine particulière dans de tels sites placés à 40—50 bp en aval du codon d'initiation de la traduction conduit à la synthèse de protéines étrangères fusionnées à l'extrémité N terminale des aminoacides bactériens dérivant de cII.

Le vecteur en cause comporte, en outre, de préférence une fonction d'antiterminaison de transcription codée par exemple par le gène N de λ noté λN. En présence du produit de transcription du gène N la transcription à partir de $P_L$ se poursuit au delà de la plupart des signaux stop. Ceci écarte les problèmes posés par un arrêt prématuré de la transcription qui peuvent se produire lorsque les gènes étrangers clonés présentent de tels signaux stop. En outre, il a été démontré que l'expression à partir de $P_L$ est améliorée dans un environnement N$^+$.

Lorsque le promoteur n'est pas $P_L$ ou $P_R$ mais $P'_R$ par exemple, la fonction d'antiterminaison peut être différente. $P'_R$ est responsable de la transcription des gènes tardifs du bactériophage λ. L'expression du gène tardif est régulée positivement par le produit du gène Q (référence 10) dont il a été démontré qu'il agit comme antiterminateur de transcription analogue à la protéine N (référence 11). Q agit à la terminaison pour l'ARN 6S de λ constitutif initié à $P'_R$ en permettant ainsi la transcription de la région du gène tardif distal. L'expression de Q est elle-même contrôlée par $P_R$ qui, comme $P_L$, est régulé par le répresseur cI. Cette cascade d'interactions peut être reconstruite sur un plasmide d'expression et utilisée de la même façon pour $P_L$.

Afin d'écarter les problèmes de toxicité et d'instabilité du système hôte-vecteur en cas de production en continu de grandes quantités d'une protéine étrangère, il est nécessaire de prévoir le contrôle de l'activité du promoteur en lui adjoignant tout ou partie d'un système d'expression inductible, en particulier thermoinductible.

De préférence, le contrôle par la température de la synthèse de la protéine étrangère est effectué au niveau de la transcription au moyen d'un répresseur thermosensible codé dans la bactérie hôte, par exemple cI857, qui réprime l'activité de $P_L$ à 28°C mais qui est inactivé à 42°C. Le répresseur agit sur l'opérateur $O_L$ qui est adjacent au promoteur $P_L$. Bien que dans le cas précédent une partie du système d'expression thermoinductible soit partie intégrante de la bactérie hôte, il est possible de prévoir que ce système fasse partie du vecteur lui-même.

Le vecteur en cause peut également comporter un gène de résistance à un antibiotique, par exemple l'ampicilline dans le cas de pBR322, mais d'autres gènes de résistance peuvent être utilisés, résistance à la tétracycline (Tet$^r$) ou au chloramphénicol (Cm$^r$).

L'incorporation d'un tel marqueur est nécessaire pour la sélection des bactéries contenant les transformants porteurs du plasmide selon l'invention pendant les expériences de clonage.

L'incorporation d'un gène de résistance permet d'augmenter la stabilité du plasmide en imposant une pression de sélection lors de la fermentation, et en outre facilite l'isolement des transformants.

Pour le clonage il est intéressant de disposer d'un système permettant de détecter l'insertion dans un plasmide d'un ADN étranger.

A titre d'exemple, il est possible de prévoir dans la zone de clonage le fragment N-terminal de la β-galactosidase de *E. coli* (lacZ')en le fusionnant avec la région d'initiation de traduction dérivée de λcII, ce qui met la traduction du fragment α sous le contrôle des séquences de cII.

Le fragment α est complémenté par l'expression du fragment ω C-terminal codé dans l'hôte, ceci conduit à une activité β-galactosidase dans les cellules. Cette activité β-galactosidase produit des colonies bleues en présence d'un substrat chromophorique, le 5-bromo-4-chloro-3-indolyl-β-D-galactosidase.

A 28°C, le promoteur $P_L$ est inactivé, le fragment α n'est pas synthétisé et les colonies demeurent blanches. Lorsque la température est amenée à 42°C, le promoteur $P_L$ est activé, le fragment α est synthétisé et les colonies virent au bleu.

L'insertion d'ADN étrangers dans les sites de clonage situés dans ce système de détection empêche la synthèse de la β-galactosidase et conduit donc à des colonies blanches aussi bien à 28°C qu'à 42°C.

Il est également possible de remplacer le gène lacZ'par d'autres gènes permettant une détection.

La présente invention concerne également des procédés permettant le clonage et l'expression de gènes codant pour des protéines particulières, notamment pour l'antitrypsine-$\alpha_1$ humaine préparée à l'état fusionné ou non fusionné, ainsi que les plasmides correspondants.

Ainsi, la présente invention concerne un procédé de clonage et d'expression d'un gène codant pour une protéine déterminée permettant de préparer ladite protéine sous forme non fusionnée, caractérisé en ce que l'on clone le gène dans un site de restriction recouvrant le codon d'initiation de la traduction d'un vecteur tel que décrit précédemment et dans lequel on reconstitue le codon d'initiation de la traduction.

L'invention concerne, en outre, un procédé de clonage d'un gène codant pour une protéine déterminée permettant de préparer ladite protéine sous forme fusionnée, caractérisé en ce que l'on clone le gène dans la zone de clonage d'un vecteur tel que décrit précédemment en aval de la séquence d'initiation de la traduction.

L'invention concerne également les vecteurs obtenus par la mise en oeuvre de ces procédés ainsi que les bactéries transformées par les différents vecteurs décrits, en particulier des bactéries gram négatives et plus particulièrement *Escherichia coli*.

L'invention concerne enfin les produits préparés par fementation des souches ainsi transformées.

Enfin, la présente invention concerne un vecteur, notamment un plasmide, comportant tout ou partie de la séquence codant pour l'antitrypsine-$\alpha_1$ humaine et notamment un vecteur tel que décrit précédemment. Parmi les plasmides comportant la séquence codant pour antitrypsine-$\alpha_1$ il faut citer le plasmide pTG922 dont la préparation est décrite dans les exemples.

Toutefois, la protéine ayant l'activité de l'anti-trypsine-$\alpha_1$ humaine préparée en mettant en oeuvre pTG922 comporte une séquence d'aminoacides fusionnée à l'extrémité N-terminale de la protéine antitrypsine-$\alpha_1$.

L'injection d'un tel produit peut dans certains cas provoquer une réponse immunitaire du patient.

Afin d'éviter cet inconvénient, il est intéressant de pouvoir exprimer, dans la bactérie, le gène naturel non fusionné de l'antitrypsine-$\alpha_1$ humaine.

Pour ce faire, dans les vecteurs de l'invention on prévoit de préférence au voisinage du codon de départ de la traduction du gène codant pour l'antitrypsine-$\alpha_1$ humaine la structure suivante:

—CAT̅A̅T̅GGAGGATCCCCAGG—
—GTATACCTCCTAGGGGTCC—

de façon que le codon de départ de la traduction soit en phase pour la traduction du gène.

Un tel plasmide permettant la préparation d'une antitrypsine-$\alpha_1$ non fusionnée peut être obtenu, par exemple, par modification du plasmide pTG922 qui conduit, lui, à la préparation d'une protéine fusionnée.

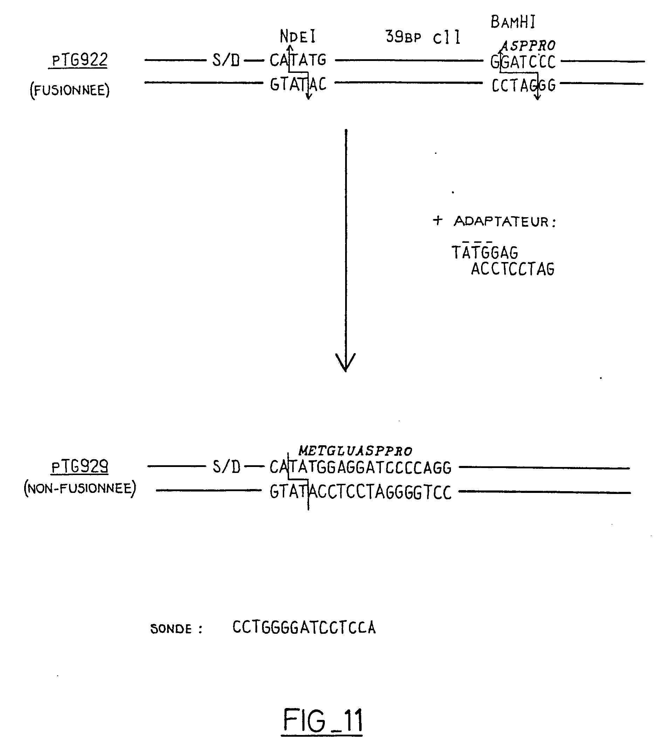

Comme cela est représenté dans la figure 11, les nucléotides codant pour l'extrémité N-terminale de la séquence cII du gène de l'antitrypsine-$\alpha_1$ fusionné dans pTG922 sont liés par des sites de restriction uniques *NdeI* et *BamHI*.

On élimine l'ADN situé entre ces deux sites par clivage du plasmide avec *NdeI* et *BamHI* puis remise en continuité du plasmide de façon que le codon de départ de la traduction soit fusionné en phase directement au début de la séquence codant pour l'antitrypsine-$\alpha_1$.

Cette remise en continuité est obtenue en utilisant un oligonucléotide adaptateur synthétique qui permet:

a) la fusion des sites *NdeI* et *BamHI*;

b) la reconstitution du codon de départ et

c) l'addition d'un codon codant pour l'acide glutamique à l'extrémité N-terminale de la protéine mature, lequel manquait dans la construction de pTG922.

D'autres plasmides ont été mis en oeuvre en particulier en utilisant dans pTG929 une séquence d'initiation de la traduction:

TCGAT̲A̲A̲C̲A̲C̲A̲ GGAACAGATCT AT̅G̅
　　*** -- ----

On obtient ainsi le plasmide pTG956.

Bien que ce plasmide permet d'obtenir une synthèse accrue d'α-AT le rendement reste relativement faible. Le fait que deux sites de fixation des ribosomes qui sont capables de conduire à une synthèse importante de protéine ne permettent pas une initiation de la traduction lorsqu'ils sont couplés au gène de l'α-AT semble indiquer que certaines caractéristiques du gène de l'α-AT lui-même limitent son expression.

C'est pourquoi on a modifié le début du gène de α-AT de la façon suivante:

| séquence originale | protéine | met | glu | asp | pro | glu | gly | asp | ala |
|---|---|---|---|---|---|---|---|---|---|
| | DNA | ATG | GAG | GAT | CCC | CAG | GGA | GAT | GCT |

| séquence mutée | protéine | met | glu | asp | pro | glu | gly | asp | ala |
|---|---|---|---|---|---|---|---|---|---|
| | DNA | ATG | GAA | GAT | CCT | CAA | GGC | GAT | GCT |
| | | | * | | * | * | * | | |

On obtient ainsi a partir de pTG 956 le plasmide pTG983 qui produit des quantités d'α-AT beaucoup plus importante.

L'invention concerne également les bactéries transformées et le produit obtenue par fermentation de ces bactéries qui sera dénommé ci-après "antitrypsine-α₁ humaine d'origine bactérienne'," pour l'antitrypsine-α₁ humaine préparée à l'état fusionné ou non fusionné, ainsi que les plasmides correspondants.

Ainsi, la présente invention concerne un procédé de clonage et d'expression d'un gène codant pour une protéine déterminée permettant de préparer ladite protéine sous forme non fusionnée, caractérisé en ce que l'on clone le gène dans un site de restriction recouvrant le codon d'initiation de la traduction d'un vecteur tel que décrit précédemment et dans lequel on reconstitue le codon d'initiation de la traduction.

L'invention concerne, en outre, un procédé de clonage d'un gène codant pur une protéine déterminée permettant de préparer ladite protéine sous forme fusionnée, caractérisé en ce que l'on clone le gène dans la zone de clonage d'un vecteur tel que décrit précédemment en aval de la séquence d'initiation de la traduction.

L'invention concerne également les vecteurs obtenus par la mise en oeuvre de ces procédés ainsi que les bactéries transformées par les différents vecteurs décrits, en particulier des bactéries gram négatives et plus particulièrement *Escherichia coli*.

L'invention concerne enfin les produits préparés par fermentation des souches ainsi transformées.

Enfin, la présente invention concerne un vecteur, notamment un plasmide, comportant tout ou partie de la séquence codant pour l'antitrypsine-α₁ humaine et notamment un vecteur tel que décrit précédemment, ainsi que les bactéries transformées et le produit obtenu par fermentation de ces bactéries qui sera dénommé ci-après "antitrypsine-α₁ humaine d'origine bactérienne".

Il doit être entendu que ce produit peut présenter des différences structurales avec l'antitrypsine-α₁ humaine sérique, mais présente néanmoins la même activité in vivo, notamment la même activité anti-élastase ou une activité équivalente par rapport à l'antitrypsine-α₁ sérique.

Par antitrypsine-α₁ humaine d'origine bactérienne, on entend définir également un produit d'origine bactérienne obtenu par fementation mais ayant ensuite été modifié chimiquement ou biologiquement.

La présente invention concerne, enfin, l'utilisation de l'antitrypsine-α₁ humaine d'origine bactérienne à titre de médicament pour le traitement et/ou la prévention des maladies humaines induites par un déficit en antitrypsine-α₁. Parmi les maladies qui peuvent être traitées ou prévenues par l'antitrypsine-α₁ selon l'invention, il faut citer les déficits en antitrypsine-α₁ hériditaires dus à un désordre génétique et qui se traduisent notamment par des emphysèmes. Il est également possible de traiter certains syndromes de difficultés respiratoires aiguës, en particulier chez les nouveau-nés et les adultes, ainsi que l'arthrite rhumatoïde, l'asthme stéroïdo-dépendant et les fibroses cystiques. Ce médicament peut également être utilisé pour remédier à certains déficits en antitrypsine-α₁ chez les fumeurs de cigarettes.

L'antitrypsine-α₁ humaine d'origine bactérienne peut être administrée par une voie quelconque, mais la voie parentérale est semble-t-il la plus satisfaisante. Toutefois, dans le cas des déficiences dues à la fumée de cigarette, l'introduction directe dans les poumons par inhalation est également très efficace.

Les dosages journaliers de l'antitrypsine-α₁ à utiliser dépendent de la nature du traitement, préventif ou curatif, ainsi que de l'importance de la déficience à traiter. Ces dosages pourront être adaptés afin de maintenir un taux déterminé d'antitrypsine-α₁ dans le sérum ou au niveau des poumons par exemple.

Les compositions pharmaceutiques correspondantes seront également adaptées à la voie d'administration et pourront être à libération contrôlée, notamment sous forme d'implant libérant lentement le principe actif dans le système circulatoire.

La présente invention comporte, bien entundu, d'autres aspects, notamment certains plasmides qui seront décrits dans les exemples ainsi que leurs mutants et dérivés et de façon générale les procédés de fermentation des bactéries transformées ainsi que les produits de ces fermentations.

D'autres caractéristiques et avantages de l'invention seront mieux compris à la lecture des exemples ci-après et des dessins ci-annexés sur lesquels:

la figure 1 représente la statégie permettant de préparer le plasmide pTG907,

la figure 2 représente la stratégie permettant de préparer le phage M13tg910,

la figure 3 représente la structure du phage M13tg910,

la figure 4 représente la stratégie permettant de préparer le plasmide pTG908,

la figure 5 représente les immunoprécipités correspondant aux produits de traduction de l'ARN total de foie humain,

la figure 6 représente une sonde isolée d'un clone de cADN humain total codant pour l'antitrypsine-$\alpha_1$,

la figure 7 représente la séquence partielle d'un clone de cADN de l'antitrypsine-$\alpha_1$ en comparaison avec la séquence publiée.

la figure 8 représente la stratégie pour préparer le plasmide pTG920,

la figure 9 représente l'analyse sur gel des protéines synthétisées par *E. coli* transformé par un plasmide portant le gène humain de l'antitrypsine-$\alpha_1$ ainsi que les immunoprécipitatés correspondants,

la figure 10 représente l'activité biologique de l'antitrypsine-$\alpha_1$ préparée à partir de E. coli.

la figure 11 représente la stratégie permettant de préparer le plasmide pTG929,

la figure 12 représente la stratégie permettant de préparer le plasmide pTG956,

la figure 13 représente les immunoprécipités des cultures marquées avec anti-sérum anti $\alpha_1$AT,

la figure 14 représente l'essai anti-élastase.

### 1) Procédés généraux

#### a) Souches bactériennes

Les souches bactériennes utilisées dans le cadre de la présente invention sont les suivantes:

.TGE900 qui est une souche de *E. coli* ayant les caractéristiques suivantes : su⁻F⁻his ilv bio ($\lambda$cl857$\triangle$Bam$\triangle$HI)

·N6437, souche de *E. coli* ayant les caractéristiques suivantes: F⁻his ilv gal⁺ $\triangle$8proC⁺ : tn10 lac$\triangle$m15 ($\lambda$cl857$\triangle$Bam$\triangle$HI).

.Jm103 qui est une souche de *E. coli* ayant les caractéristiques suivantes: $\triangle$(lac-pro) sup^E thi endA sbcB15 sttA rK⁻ nK⁺ /F¹ traD36 proAB⁺ laci^a lacZ$\triangle$m15.

Les souches mentionnées précédemment ont été utilisées parce qu'elles étaient disponibles, mais il est bien entendu qu'il est possible d'utiliser d'autres souches dans la mesure où elles présentent certaines caractéristiques essentielles qui sont rappelées dans le cours de la description détaillée.

#### b) Préparation des ADN

Des mini-préparations d'ADN de plasmides ou de phages M13 sont effectuées comme cela est décrit par Ish-Horowitz (référence 12) à la seule différence que l'ADN est précipité une seconde fois avec de l'éthanol avant d'être utilisé.

Les maxi-préparations sont effectuées comme cela est décrit dans la publication précédente, ave une purification complémentaire par un gradient de densité CsCl/bromure d'éthidium.

#### c) Techniques de clonages

Le traitement des ADN avec des enzymes de restriction est effectué, sauf indications contraires, en utilisant les conditions indiquées par le fabricant (New England Biolabs, Bethesda Research Laboratories et Boehringer Mannheim).

Lorsque cela est nécessaire, les phosphates des extrémités 5' sont éliminés en utilisant, soit une phosphatase alcaline bactérienne, soit une phosphatase d'intestin de veau, pendant 30 minutes à 37°C dans le tampon d'enzyme de restriction.

La réparation des extrémités cohésives utilisant la polymérase de Klenow (Boehringer Mannheim) est effectuée à 25°C pendant 15 minutes dans un mélange de 50 mMol Tris HCl, pH 7,8, 5 mMol MgCl₂, 10 mMol de β-mercaptoéthanol, 0,4 mMol de dNTPs avec l'enzyme et 10 à 200 µg/ml d'ADN. ·

La nucléase S₁ (Miles) est utilisée à 2 u/µg d'ADN à 25°C pendant 30 minutes dans un milieu 0,3 Mol NaCl, 0,03 Mol NaOAc, pH 4,8, 0.003 Mol ZnCl₂.

Bal31 est utilisée selon le procédé de Panayotatos et al. (référence 13). Les ligations sont effectuées à 15°C (sauf lorsque cela est indiqué différemment) pendant 4 à 24 heures en utilisant de l'ADN ligase T₄ (Boehringer Mannheim) avec 100 mMol de NaCl, 66 mMol de Tris HCl, pH 7,5, 10 mMol de MgCl₂, 0,5 mMol de spermidine, 0,2 mMol de EDTA, 2 mMol de DTT, 1 mMol d'ATP, 0,1 mg/ml de BSA et 5 à 50 µg/ml d'ADN.

Pour la ligation d'extrémités cohésives, on utilise environ 30 unités/ml de ligase. Pour la ligation des extrémités franches on utilise environ 100 unités/ml de ligase.

Entre les différentes réactions enzymatiques, des échantillons d'ADN sont extraits avec un mélange phenol/chloroforme puis précipités à l'éthanol. Lorsque cela est nécessaire, du tARN de *E. coli* ou de levures est utilisé comme entraîner. Les adaptateurs moléculaires (Collaborative Research, Bethesda Research Laboratories, New England Biolabs) sont préhybridés et utilisés en excès molaire de 10 à 50 fois pour les extrémités franches d'ADN utilisant les conditions de tampon décrites précédemment avec 100 unités/ml de ligase T₄ à 4°C pendant 15 heures. Lorsque l'on utilise des adaptateurs non phosphorylés, les adaptateurs qui n'ont pas réagi sont éliminés, directement après ligation par précipitation avec le tétrachlorhydrate de spermine (Hoopes et al. — référence 14).

Lorsque l'on utilise des adaptateurs phosphorylés, le mélange de ligation est tout d'abord extrait avec un mélange phénol/chloroforme puis précipité avec de l'éthanol avant coupure spécifique avec les enzymes de restriction appropriées puis précipitation avec le tétrachlorhydrate de spermine.

Les cellules bactériennes compétentes sont préparées puis transformées avec les plasmides ou transfectées par l'ADN de M13 selon les procédés décrits par Dagert et Ehrlich (référence 15).

Exemple 1

L'exemple de synthèse de vecteurs selon la présente invention qui sera décrit ci-après n'est bien entendu nullement limitatif et il est possible d'obtenir des vecteurs entrant dans le cadre de la présente invention en procédant de façon distincte.

La préparation d'un vecteur selon l'invention implique tout d'abord la préparation d'un plasmide comportant:

l'origine de réplication de pBR322,

le gène de résistance à l'ampicilline de ce même plasmide amp$^R$,

le promoteur P$_L$ et le gène λN, et, en outre, au cours de cette synthèse on doit s'efforcer de faire disparaître les sites de restriction gênants afin de pouvoir obtenir plus tard dans le cours de la synthèse des sites uniques de restriction.

1) Suppression du site PstI dans pBR322

Le plasmide de base utilisé est le plasmide pBR322; toutefois, celui-ci présente l'inconvénient d'avoir à l'intérieur du gène amp$^R$ un site de restriction PstI, car un site de même nature sera utilisé par la suite dans la zone de clonage comme site unique de restriction. Il convient donc de faire disparaître ce site de restriction PstI en utilisant un mutant du plasmide pBR322, le plasmide pUC8, dans lequel le gène de résistance à l'ampicilline ne présente pas de site de restriction PstI (ce site a été éliminé par mutation in vitro). pBR322 est commercialisé notamment par Bethesda Research Laboratories et pUC8 est décrit dans l'article référencé 16.

Pour ce faire, on échange le fragment PvuI/PvuII de 1 669 bp de pBR322 avec le fragment analogue PvuI/PvuII du plasmide pUC8. Afin de réaliser cet échange les plasmides pBR322 et pUC8 sont traités sucessivement par pvuI, PvuII, puis circularisés par section d'une ligase.

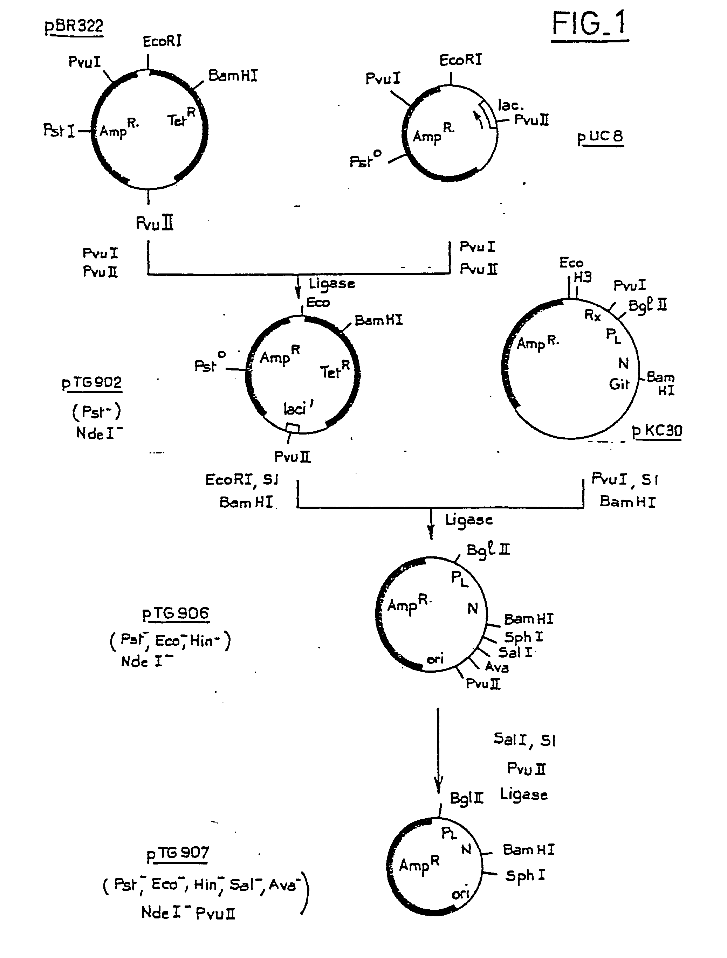

On obtient ainsi le plasmide pTG902 qui ne présente plus de site de restriction PstI et qui a également perdu le site de restriction NdeI présent à l'origine sur pBR322 (non représenté sur la fig. 1). En outre, le plasmide pTG902 porte un fragment de 50 bp correspondant à la séquence lacI' dans lequel se trouve le site PvuII.

2) Insertion du promoteur P$_L$ et du gène λN et préparation du plasmide de pTG907

Le promoteur P$_L$ et le gène λN sont isolés du plasmide pKC30 pour être insérés dans pTG902, le segment prélevé contient également l'opérateur O$_L$ sur lequel agira le répresseur thermosensible cI850, comme cela sera décrit dans les essais. En outre, le procédé permet de supprimer les sites EcoRI et HindIII tout en conservant un site unique BamHI en aval du gène N pour pouvoir ensuite insérer λcIIrbs. Les plasmide de pKC30 est décrit dans la référence 17.

pTG902 est coupé en son site de restriction unique EcoRI et les extrémités 5' sortantes sont éliminées par traitement à la nucléase S$_1$. Après une digestion avec BamHI, le fragment le plus important est purifié sur gel.

Le fragment portant le promoteur P$_L$ et le gène λN est préparé de la même façon à partir de pKC30 en traitant successivement le plasmide par PvuI, la nucléase S$_1$, et BamHI. Les fragments, après purification sur gel, sont soumis à l'action de la ligase, ce qui conduit à la fusion EcoRI/PvuI et à la reconstitution du site BamHI.

Le mélange de ligation est utilisé pour transformer des cellules hôtes compétentes, TGE900, à 30°C. Cette souche contient le prophage λ délété, λcI857△Bam△HI, qui fournit le répresseur de λ thermosensible, cI857, qui est nécessaire pour bloquer la transcription à partir de P$_L$.

Ceci est important car l'activité de P$_L$ dans un milieu N$^+$ est léthale compte tenu de la fonction d'antiterminaison de N.

Après analyse par enzymes de restriction, les clones contiennent des plasmides de structure correcte et sont ensuite testés pour leur manque de viabilité à 42°C.

L'un des plasmides obtenus, pTG906, est traité de façon à éliminer le segment PvuII-SalI de manière à supprimer les sites de restriction compris sur ce segment et afin de faire disparaître également les deux sites de restriction extrêmes. Pour ce faire, pTG906 est traité successivement avec SalI, la nucléase S$_1$, PvuI et la ligase.

On obtient ainsi le plasmide pTG907 qui comporte l'ensemble des éléments évoqués au début de cette étape et, en outre, qui est Pst$^-$ Eco$^-$ Hind$^-$ Sal$^-$ Ava$^-$ Nde$^-$ PvuII$^-$. La synthèse de ce plasmide est représentée sur la figure 1.

3) Clonage de la région λcIIrbs

La seconde phase importante de la synthèse consiste à insérer la région λcIIrbs sous forme d'un fragment AvaI/TaqI dans le début du gène lacZ' (fragment α de la β-galactosidase) lequel a été cloné dans le phage M13 nommé M13tg110. Cette stratégie permet un test fonctionnel simple pour rbs, à savoir la production de la protéine lacZ' et, par conséquent, d'obtenir des plaques bleues en présence d'IPTG et de Xgal; ceci permet également un séquençage rapide de la construction en utilisant la méthode dite du didéoxy.

Dans le cours des expériences, différents dérivés du phage M13 seront mentionnés. Précédemment on

a mentionné le phage M13tg110 et dans la suite de la description on utilisera d'autres phages du même type dont la construction va être rappelée ci-après.

La construction de ces vecteurs est indiquée dans le tableau I ci-après sur lequel figurent les références du phage de départ, la nature de l'enzyme de restriction avec laquelle il a été découpé, le traitement particulier qu'ont subi les fragments ainsi obntenus et la nature de l'insert qui a été fixé dans les sites ainsi révélés.

Le phage M13mp7 est commercialisé notamment par la société Bethesda Research Laboratories.

M13mp701 est obtenu à partir de M13mp7 par remplacement du fragment PstI/EcoRI à droite (CTG CAG . . . GAA TTC) par la séquence: CTG CAG CAA TTC.

Le principe de la construction de M13tg110 mettant en jeu AccI, SalI et un adapteur HindIII est similaire à celui décrit dans le schéma suivant:

ATG ACC ATG ATT ACG AAT TCC CCG GAT CCG TCG ACC TGC AGC AAT TCA CTG GCC          M13sp701

Bam HI

ATGACCATGATTACGAATTCCCCG                    GATCCGTCGACCTGCAGCAATTCACTGGCC

TACTGGTACTAATGCTTAAGGGGCCTAG                GCAGCTGGACGTCGTTAAGTGACCGG

DNA polymerase

ATGACCATGATTACGAATTCCCCGGATC                GATCCGTCGACCTGCAGCAATTCACTGGCC

TACTGGTACTAATGCTTAAGGGGCCTAG                CTAGGCAGCTGGACGTCGTTAAGTGACCGG

Linker HindIII

ATGACCATGATTACGAATTCCCCGGATCCCAAGCTTGG        CCAAGCTTGGGATCCGTCGACCTGCAGCAATTCACTGGCC

TACTGGTACTAATGCTTAAGGGGCCTAGGGTTCGAACC        GGTTCGAACCCTAGGCAGCTGGACGTCGTTAAGTGACCGG

HindIII

ATGACCATGATTACGAATTCCCCGGATCCCA             AGCTTGGGATCCGTCGACCTGCAGCAATTCACTGGCC

TACTGGTACTAATGCTTAAGGGGCCTAGGGTTCGA          ACCCTAGGCAGCTGGACGTCGTTAAGTGACCGG

T$_4$ DNA ligase

ATG ACC ATG ATT ACG ATT TCC CCG GAT CCC AGG CTT GGG ATC CGT CGA CCT GCA GCA ATT CAC TGG CC

Eco RI          Bam HI          Hind III          Bam HI          Sal I          PstI

TABLEAU I

| Nom | Phage parental | Site de clivage | Traitement | Insert | Phase de HindIII | Phase de BamHI | Phase de PstI | Complémentation de lacα +/− |
|---|---|---|---|---|---|---|---|---|
| M13mp701 | M13 mp7 | Construction D. R. Bentley | | | O | I | I | + |
| M13tg102 | M13mp701 | AccI | ADN polymérase | séquence HindIII (a) | I | I | I | + |
| M13tg110 | M13mp701 | AccI | S₁ nucléase | séquence HindIII (a) | III | I | II | − |
| M13tg115 | M13tg110 | EcoRI | S₁ nucléase | séquence BglII (b) | II | III | I | + |

(a) Adaptateur de séquence HindIII phosphorylé CCAAGCTTGG
(b) Adaptateur de séquence BglII non phosphorylé CAGATCTG

EP 0 114 777 B1

Les protocoles d'utilisation des enzymes (restriction, ADN polymérase, T₄ADN ligase) sont ceux indiqués dans les notices des fournisseurs. Le "linker" HindIII est un oligonucléotide synthétique de séquence $^5_P$" CCAAGCTTGG $^3$' (Collaborative Research Inc., Waltham, Mass 02154, E.U.A.).

La région cIIrbs est isolée à partir d'un plasmide pOGII (réf 18) qui contient un fragment de λHaeIII/Sau3A qui s'étend du milieu du gène cro jusqu'au milieu de la région codant pour cII (cro et cII étant impliqués notamment dans la régulation de la lysogénie du bactériophage λ).

On prélève dans pOG11 le fragment Aval/TaqI de 186 bp contenant le région cIIrbs et on le traite avec la polymérase Klenow. D'autre part, on traite le phage M13tg110 par BamHI puis par la polymérase de Klenow suivi d'un traitement à la phosphatase intestinale de veau. Les fragments obtenus sont soumis à l'action de la ligase T₄.

Un examen des séquences du fragment de pOG11 comportant la région cIIrbs et du phage M13tg110 permet de prévoir que l'insertion du fragment portant cIIrbs dans le site BamHI de M13tg110 doit conduire à l'obtention de plaques bleues après fermentation des bactéries transfectées compte tenu du fait que le gène lacZ' est en phase pour la traduction à partir de AUG du gène de cII, tandis que les plaques correspondant à la souche parentale M13tg110 sont blanches.

Les plaques bleues sont prélevées puis les colonies sont sélectionnées par analyse de restriction enzymatique des mini-préparations et l'on vérifie ensuite par séquençage que la construction obtenue est correcte.

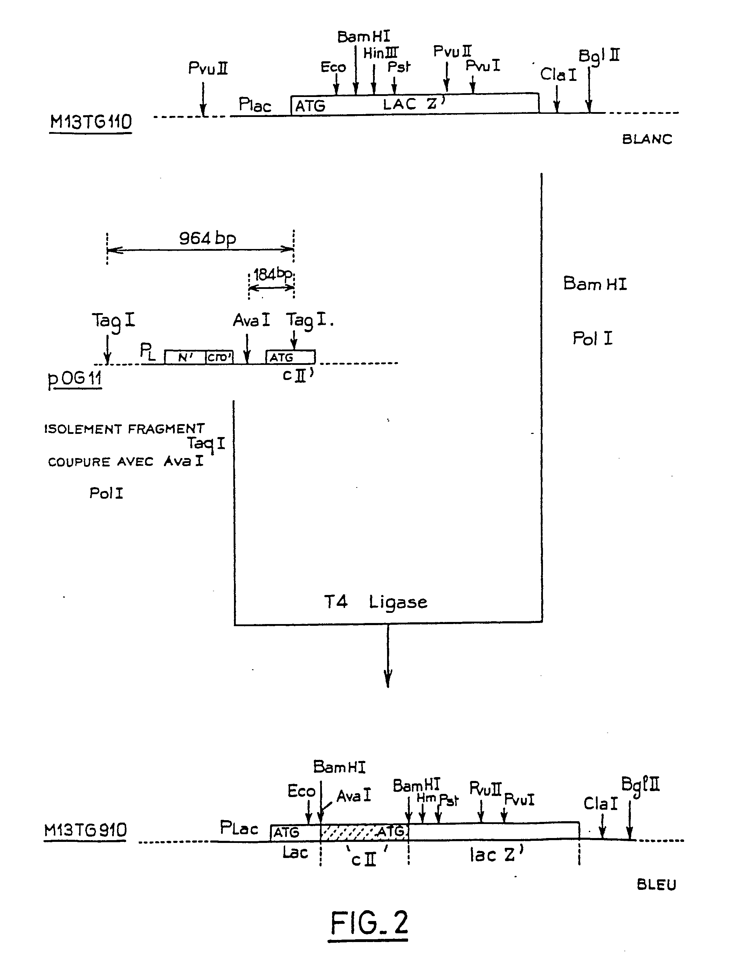

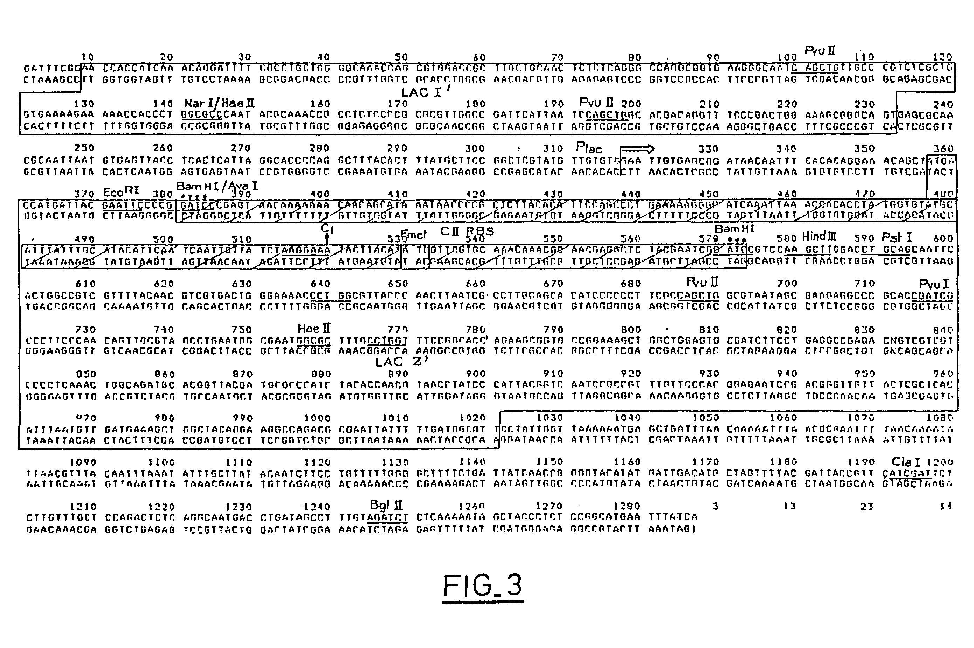

On obtient ainsi un clone résultant M13tg910 dont la structure globale est représentée à la partie inférieure de la Figure 2 et la structure détaillée est représentée à la figure 3.

On constate que l'insertion du fragment cIIrbs reconstruit en amont les sites BamHI et Aval et en avail le site BamHI.

La traduction à partir de AUG de cII conduit à la fusion des 13 aminoacides terminaux de cII aux 8 aminoacides du NH₂ terminal de la protéine lacZ'.

4) Insertion du fragment λcIIrbs dans le plasmide pTG907

La troisième étape de cette synthèse consiste à transférer le fragment cIIrbs/lacZ' du phage M13tg910 sur le plasmide vecteur pTG907 préparé précédemment.

Pour ce faire, il convient tout d'abord d'eliminier les sites EcoRI, BamHI et Aval en amont de cIIrbs puis d'insérer un site BglII.

Dans ces conditions, cIIrbs peut être prélevé sous forme d'un fragment BglII-BglII et placé dans le site BamHI en aval du promoteur P$_L$ et du gène λN de pTG907.

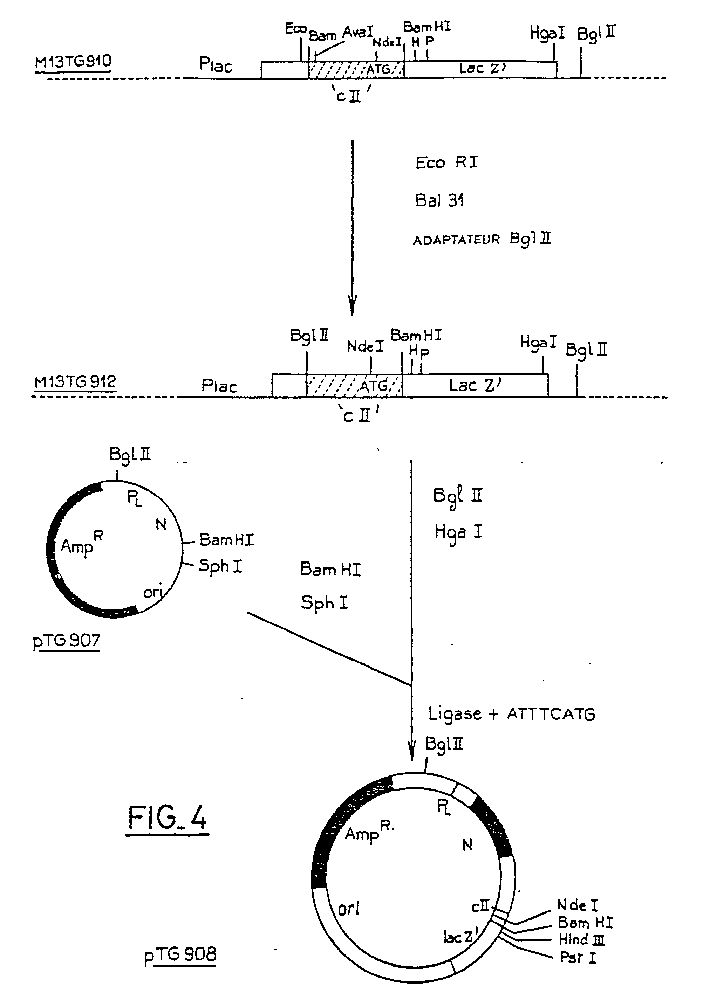

Le phage M13tg910 est digéré avec EcoRI puis traité avec Bal31 puis ensuite par la polymérase de Klenow. Les fragments obtenus sont alors soumis à l'action de la ligase en présence d'adaptateurs BglII non phosphorylés. Le mélange de ligation obtenu est utilisé pour transformer des cellules compétentes JM103. On sélectionne alors les plages bleues. Ces clones sont ensuite analysés afin de vérifier qu'ils contiennent le site BglII et qu'ils ne présentent plus de site EcoRI ou BamHI en amont. On obtient ainsi des clones tels que M13tg912 dont la structure est représentée sur la Figure 4.

Le traitement par Bal31 a produit une délétion de 101 bp éliminant les sites EcoRI, BamHI et Aval; ainsi que les séquences de lac ATG et lac Shine/Dalgarno. Le site BglII introduit se trouve placé environ 100 bp en amont de l'ATG de cII et 10 bp en aval de P$_{lac}$.

Comme cela a été dit précédemment, il était envisagé de transférer le fragment BglII-BglII comportant cIIrbs et lacZ' de M13tg912 dans le site BamHI de pTG907 préparé à l'origine. Toutefois cette construction n'a pas été possible car elle se révèle léthale pour les souches hôtes, c'est pourquoi il a fallu mettre au point une stratégie différente mettant en oeuvre un adaptateur Hgal/SphI qui permet l'insertion d'un fragment BglII/Hgal portant cIIrbs et lacZ' entre les sites BamHI et SphI de pTG907.

Le fragment BamHI/SphI de pTG907, le fragment BglII/HpaI portant cIIrbs et lacZ' et l'adaptateur phosphorylé ont été préhybridés dans un rapport molaire de 1:2:1 puis traités avec la ligase T₄. Des aliquots sont utilisés pour transformer les cellules compétentes de la souche 6150 à 30°C.

Les cellules intéressants sont identifiées en sélectionnant les transformants avec un fragment cIIrbs/lacZ' marqué au P³² et la construction obtenue est confirmée par une étude de restriction enzymatique.

Afin d'avoir une première indication montrant que les différents éléments du système d'expression se conduisent comme cela est désiré, le plasmide obtenu, pTG908, est transféré dans une souche hôte N6437 qui possède à la fois c1857 et le fragment ω de la β-galactosidase complémentant le fragment α qui est codé par le plasmide.

Les transformants obtenus placés sur une boîte contenant IPTG + Xgal sont blancs à 28°C puis virent au bleu environ 30 minutes après lorsqu'on les transfère à 42°C.

## Exemple 2

De la même façon que précédemment, il est possible d'utiliser un autre promoteur que P$_L$.

pBλH₃ construit par V. Pirotta comporte le gène cl857, P$_R$ et une partie du gène cro cloné dans pBR322.

Ce clone contient un site BglII dans le gène cro' en aval du promoteur P$_R$ (promoteur de droite) qui est sous le contrôle du cl857 codé par le plasmide.

Le gène Q, $P'_R$, le gène 6S ARN et une partie du gène S tardif sont contenus dans un fragment λBcII (référence 19). Ce fragment peut être inséré dans le site BgIII de pBλH3 en mettant ainsi le gène Q sous le contrôle de $P_R$ du plasmide. $P'_R$ plus l'ensemble de système de contrôle en cascade sera alors contenu dans un fragment EcoRI qui peut être utilisé pour remplacer $P_L$ et N qui se trouvent placés dans un fragment BgIII/AvaIII dans pTG908. Ceci conduit à une construction analogue de pTG908 mais dans lequel la transcription est contrôlée par $P'_R$.

Les exemples suivants concernent le clonage du gène codant pour l'antitrypsine-$α_1$.

Pour ce faire, on a isolé un clone de cADN codant pour la séquence complète du mARN codant l'antitrypsine-$α_1$ humaine. On a inséré cette séquence dans un vecteur d'expression plasmidique bactérien préparé précédemment et démontré la synthèse dans *E. coli* d'une grande quantité de polypeptides qui présentent une réaction avec un anticorps correspondant à l'antitrypsine-$α_1$ humaine naturelle et qui présentent, in vitro, l'activité biologique de l'antitrypsine-$α_1$ et sa capacité à inhiber l'activité de l'élastase. Cette antitrypsine-$α_1$ produite à partir de bactéries constitue la base d'un produit qui peut être utilisé dans une thérapie de remplacement pour le traitement des désordres qui comportent une déficience en antitrypsine-$α_1$.

<div align="center">

Exemple 3

Clonage et isolement d'un cADN complet condant pour l'antitrypsine-$α_1$ humaine et son expression dans E. coli

</div>

1) Clonage du cADN de l'antitrypsine-$α_1$

*a) Isolement du polyA$^+$ mARN de foie humain et détection de l'activité de mARN antitrypsine-$α_1$*

Les foies humains sont obtenus post mortem et rapidement congelés dans l'azote liquide. L'ARN est préparé à partir de 5 g de foie en utilisant le procédé au chlorhydrate de guanidine tel que décrit dans la référence 20. L'ARN est chromatographié sur une colonne poly U-Sépharose (Pharmacia) pour enrichir en polyA$^+$ mARN.

L'ARN total et le polyA$^+$ mARN sont traduits dans un lysat de réticulocytes de lapin traité par la nucléase (BRL) en utilisant les conditions décrites précédemment dans la référence 20. De façon à étudier les modifications post-traductionnelles, des membranes de pancréas de chien (réf. 21) sont ajoutées au système de traduction. Les immunoprécipitations, en utilisant un antisérum antitrypsine-$α_1$ commercial (Nordic Immunology), sont décrites dans la référence 22.

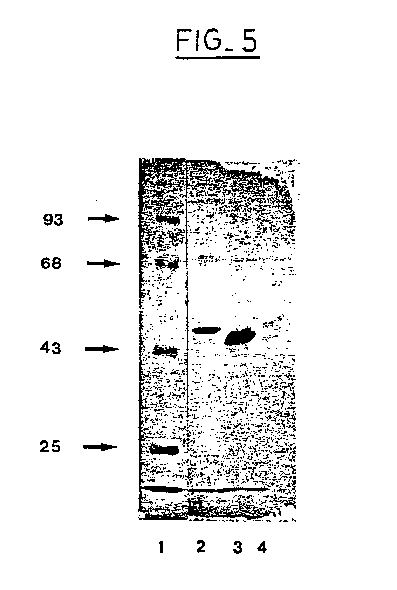

La figure 5 représente les immunoprécipités correspondant aux produits de traduction de l'ARN total de foie humain. Ces ARN ont été traduits dans les lysats de réticulocytes de lapin traités par la nucléase à 300 µg/ml, en présence ou en l'absence de membranes microsomales de pancréas de chien. La méthionine $S^{35}$ a été utilisée comme traceur radioactif. Les aliquots contenant environ $10^6$ cpm de radioactivité insoluble au TCA sont soumis à une électrophorèse sur un gel à 10% de polyacrylamide SDS, puis fluoro-graphiés et autoradiographiés.

La ligne 1 représente les protéines marqueurs radioactives, la taille étant indiquée en $10^3$ daltons;

La ligne 2 représente les immunoprécipités avec l'antisérum antitrypsine-$α_1$ en présence de membranes;

La ligne 3 est identique à la ligne 2 mais les produits obtenus ayant été préparés en l'absence de membranes;

la ligne 4, comme pour la ligne 3 mais en utilisant un antisérum non immune.

L'immunoprécipitation du produit de traduction d'ARN total de foie humain avec l'antisérum antianti-trypsine$α_1$ indique qu'un polypeptide spécifique de poids moléculaire de 45 000 daltons est précipité lorsque la traduction est effectuée en l'absence de membranes microsomales. L'addition de membranes microsomales à la traduction conduit à un polypeptide immunoprécipité légèrement plus grand (48 000 daltons). Cette augmentation de la taille du produit de traduction du mARN de l'antitrypsine-$α_1$ en présence de membranes microsomales est probablement due à une modification post-traductionnelle telle que la glycosylation. Les polypeptides immunoprécipités de petite et de grande taille sont mis en compétition par l'addition d'antitrypsine-$α_1$ humaine native non marquée et confirme l'identité des produits de traduction. Ainsi, un ARN messager de l'antitrypsine-$α_1$ biologiquement actif peut être facilement détecté à la fois dans l'ARN total et dans le mARN poly A$^+$ préparé à partir de foie humain; la quantité de mARN α-AT est de l'ordre de 1 à 5% dans le foie.

*b) Synthèse de cADN et préparation d'une banque de clones de cADN de foie humain*

Le poly A$^+$ mARN est utilisé comme matrice pour une transcription reverse en utilisant comme amorce un oligomère $(dT)_{12-18}$. L'ADN complémentaire est synthétisé dans 100 µl de milieu réactionnel contenant 100 mMol. Tris HCl, pH 8,3, 50 mMol. de KCl, 8 mMol. $MgCl_2$, 30 mMol. β-mercaptoéthanol, 1 µg d'oligo $(dT)_{12-18}$, 5 µg de poly A$^+$ mARN, 500 µMol. dATP, dCTP, dGTP, dTTP, et 80 unités de transcriptase reverse de virus de myeloblastose aviaire (Life Sciences Inc., St Petersberg, Floride).

Après incubation à 42°C pendant 45 minutes, la réaction est terminée et le complexe cADN est dénaturé en chauffant à 100°C pendant 3 minutes et transfert rapide sur bain de glace.

Pour la synthèse du second brin d'ADN, le mélange réactionnel du premier brin est dilué 5 fois et ajusté à une concentration finale de 100 mMol. Hepes-KOH, pH 6,9, 100 mMol. KCl, 200 µMol. dATP, dGTP, dTTP, 200 µMol. de dCTP $P^{32}$, l'activité spécifique étant de 0,5 Ci/mmol., 10 unités du fragment Klenow de l'ADN polymérase de *E. coli* (Boehringer Mannheim).

<div align="center">15</div>

L'incubation est effectuée à 25°C pendant 2 heures. Le rendement en cADN bicatenaire (dscADN) tel que cela est mesuré par la radioactivité est de 970 ng. Le mélange réactionnel est extrait avec un volume égal de phénol:chloroforme (50:50) saturé avec 10 mMol. Tris HCl, pH 7,5, 1 mMol. EDTA et le cADN est précipité par l'éthanol.

Le dscADN est rendu à extrémité franche par une digestion avec 5 unités de nucléase S1 dans un volume de réaction de 100 µl contenant 30 mMol. d'acétate de sodium, pH 4,8, 300 mMol. de NaCl, 3 mMol. $ZnCl_2$.

Après 1 heure à 37°C, on ajoute de l'EDTA et du SDS jusqu'à une concentration finale 10 mmol. et 0,1% respectivement puis le mélange réactionnel est chauffé à 65°C pendant 10 minutes. Le dscADN traité par S1 est ensuite appliqué sur un gradient de sucrose linéaire 5—20% dans un tampon 100 mMol. Tris HCl, pH 7,5. 100 mMol. NaCl, 5 mMol. EDTA puis centrifugé à 30 000 rpm à 15°C pendant 15 heures avec un rotor Beckman SW60 Ti. Des fractions de 0,5 ml sont collectées et la quantité de cADN dans chacune des fractions est déterminée en mesurant la radioactivité insoluble-TCA dans les aliquots de chacune des fractions. La taille du dscADN dans chacune des fractions est mesurée en analysant des aliquots sur un gel d'agarose neutre suivi par une autoradiographie. Les fractions contenant le dscADN d'un poids moyen de 1 kb et plus sont rassemblées, 5 µg de tARN de E. coli sont ajoutés comme entraineur et les acides nucléiques sont récupérés par précipitation à l'éthanol, suivie par une centrifugation.

Le dscADN est dissous dans un petit volume de 1/10 × SSC (15 mMol. NaCl, 1,5 mMol. de citrate de sodium, pH 7,0).

100 ng de cADN bicatenaire sont allongés à l'extrémité 3' avec une extension dC homopolymère par incubation dans un milieu réactionnel de 30 µl contenant 140 mMol. de cacodylate de sodium, pH 6,8, 1 mMol. de chlorure de cobalt, 0,1 mMol. de dithioérithritol, 100 µMol. dCTP $H^3$, activité spécifique 6,7 Ci/mmol. et 5 unités de transférase déoxynucléotide terminale (BRL) pendant 15 minutes à 37°C.

Des petits morceaux dG homopolymères (approximativement 13 éléments par extrémité 3') sont ajoutés dans le même milieu réactionnel au plasmide pBR322 digéré au préalable avec l'enzyme Pstl. Le plasmide pBR322 ainsi obtenu est ensuite purifié à partir du mélange réactionnel par extraction au phénol suivie par une électrophorèse préparative et une électro-élution sur un gel d'agarose à 1%.

Le dscADN obtenu directement du milieu réactionnel est mélangé avec pBR322-dG dans un rapport de 15 ng de cADN pour 500 ng de vecteur dans 150 µl de 10 mMol. Tris HCl, pH 7,5, 100 mMol. NaCl, 1 mMol. EDTA, chauffé à 65°C pendant 10 minutes puis incubé à 42°C pendant 2 heures. Le mélange de réaction est ensuite utilisé pour transformer (référence 23) une souche de E. coli 1106, les transformants sont sélectionnés sur plaque d'agar LB contenant 15 µg/ml de tétracycline. Un total de 15 000 recombinants sont obtenus à partir de 30 ng de dscADN qui, approximativement pour 50% d'entre eux, contiennent des insertions de cADN supérieures à 1 kb. Tous les transformants sont prélevés des plaques, suspendus dans un milieu LB contenant 15 γ/ml de tétracycline et la banque est stockée à — 20°C après addition de glycérol jusqu'à 50%.

c) *Stratégie pour isoler les clones de cADN de l'antitrypsine-$\alpha_1$*

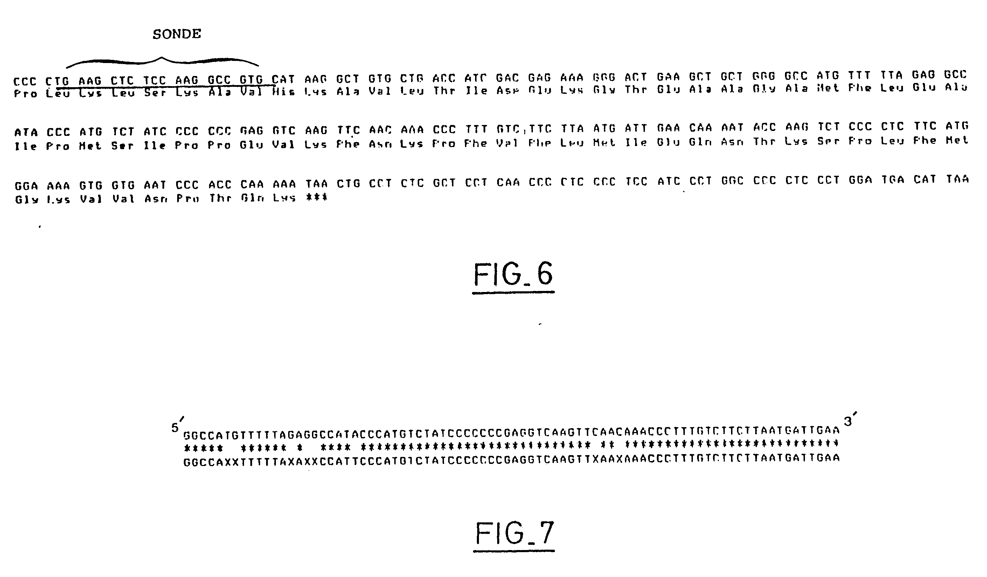

La séquence de 306 pb du clone de cADN d'antitrypsine-$\alpha_1$ humaine incomplète codant les 69 aminoacides terminaux et incluant 66 nucléotides de la séquence non codante en 3' (référence 24) est analysée par ordinateur de façon à déterminer la séquence d'oligonucléotides qui doit être utilisée comme sonde pour un clone de cADN d'antitrypsine-$\alpha_1$. Ceci est effectué en divisant les 306 paires de base en tous les groupements possibles de 21 bases (21mers) qui sont ensuite analysés séparément pour leur possibilité de former une structure double brin intramoléculaire (épingle à cheveux). Le 21mer choisi, 5'-TGAAGCTCTCCAAGGCCTGTG-3' (qui est représenté à la fig. 6) ne contient aucune épingle à cheveux plus longue que 2 pb. Le complément de cette séquence, c'est-à-dire 5'-GCACGGCCTTGGAGAGCTTCA-3', est synthétisé sur un support de gel de silice comme cela a été décrit précédemment (référence 25). Après avoir marqué l'extrémité 5', la taille de l'oligonucléotide est testée électrophorèse sur gel de polyacrylamide et la séquence est déterminée par la méthode de Maxam et Gilbert.

d) *Analyse de la banque cADN de foie humain*

Environ 15 000 colonies sont mises en croissance pendant une nuit sur des boîtes d'agar LB contenant 15 µg/ml de tétracycline. Le jour suivant, les colonies bactériennes sont transférées sur papier Whatman 540 (référence 26). Le bactéries demeurant sur les boîtes d'origine contenant la tétracycline sont remises en croissance pendant quelques heures à 37°C.

Afin de préparer l'ADN plasmidique bactérien pour l'hybridation, les filtres sont lavés pendant 5 minutes avec NaOH 0,5 Mol. Après séchage à l'air les filtres sont neutralisés par un lavage séquentiel dans un tampon 0,5 Mol. Tris HCl, pH 7,5 2 × SSC. Les filtres sont alors rincés avec de l'alcool et séchés à l'air. Les débris bactériens restants sont éliminés par digestion pendant 2 heures à 37°C avec 25 µg/ml de protéinase K dans 50 mMol. Tris HCl, pH 7,5 5 mMol. EDTA, 0,5% SDS.

La sonde d'hybridation est préparée par marquage de l'extrémité 5' avec la polynucléotide kinase (PL Biochemicals) sur 80 ng du 21mer avec 50 uCi de $P^{32}$-γ-ATP. L'hybridation est effectuée à 37°C pendant une nuit dans 40 ml de 6 × SSC, 1 × solution de Denhardt, 10 mMol. de phosphate de sodium pH 7,5, 50% de formamide. Les filtres sont alors lavés à la température de la pièce plusieurs fois avec 6 × SSC, 0,1% SDS.

Les filtres sont séchés et autoradiographiés pendant une nuit. Des signaux positifs très forts sont clairement détectables au delà du bruit de fond de l'hybridation.

e) *Identification des plasmides recombinants contenant le cADN de l'antitrypsine-$\alpha_1$*

Les colonies bactériennes correspondant aux signaux positifs importants sont déterminées en orientant l'autoradiographe avec les boîtes d'origine contenant la tétracycline. Les ADN plasmidiques sont préparés à partir de petites cultures de chacune des colonies bactériennes (référence 27) et la taille de l'insert de cADN de chaque plasmide est déterminée par digestion avec PstI. Un plasmide appelé pTG603 se trouve contenir un insert d'environ 1,6 kb possédant deux sites AvaI et un site unique BamHI. La taille des fragments produits par double digestion avec AvaI + PstI (250 paires de base) est en accord avec ce qui était prévu pour un clone de l'antitrypsine-$\alpha_1$ ayant une longueur complète (références 24, 28). Pour confirmer que ce plasmide contient bien en fait l'insert de cADN de l'antitrypsine-$\alpha_1$, l'insert est transféré sous forme de fragment PstI sur le phage M13mp8 après découpage avec PstI puis séquencé en utilisant des didéoxynucléotides comme terminateurs de chaîne. La séquence obtenue (fig. 7) confirme l'identité du cADN avec celui de l'antitrypsine-$\alpha_1$. La séquence obtenue est comparée à la région correspondante connue pour l'ADN humain codant pour l'antitrypsine-$\alpha_1$. A la partie supérieure de la figure 7 figure la séquence partielle du clone de cADN de l'antitrypsine-$\alpha_1$ complète et à la partie inférieure figure la séquence partielle de pTG603 telle quelle a été déterminée. L'homologie entre les deux séquences de nucléotides est représentée par des astérisques. X représente des nucléotides qui n'ont pas été déterminés.

2) Expression du cADN de l'antitrypsine-$\alpha_1$ humaine dans *E. coli*

Le clone de cADN de l'antitrypsine-$\alpha_1$ humaine pTG603 contient un site de restriction unique BamHI immédiatement après le codon pour le premier aminoacide de la protéine mature. La capacité de codage pour le polypeptide mature entier, à l'exception de l'acide glutamique initial, est ainsi contenu dans un fragment BamHI/PstI qui a été cloné sur le vecteur d'expression pTG920; dans cette construction, la transcription est effectuée à partir du promoteur de λ à gauche, $P_L$, et la traduction est initiée à l'ATG de λcIIrbs qui, accompagné par le site de fixation des ribosomes et les 39 premiers pb du gène λcII, sont fusionnés au début du gène lacZ' comme cela est indiqué.

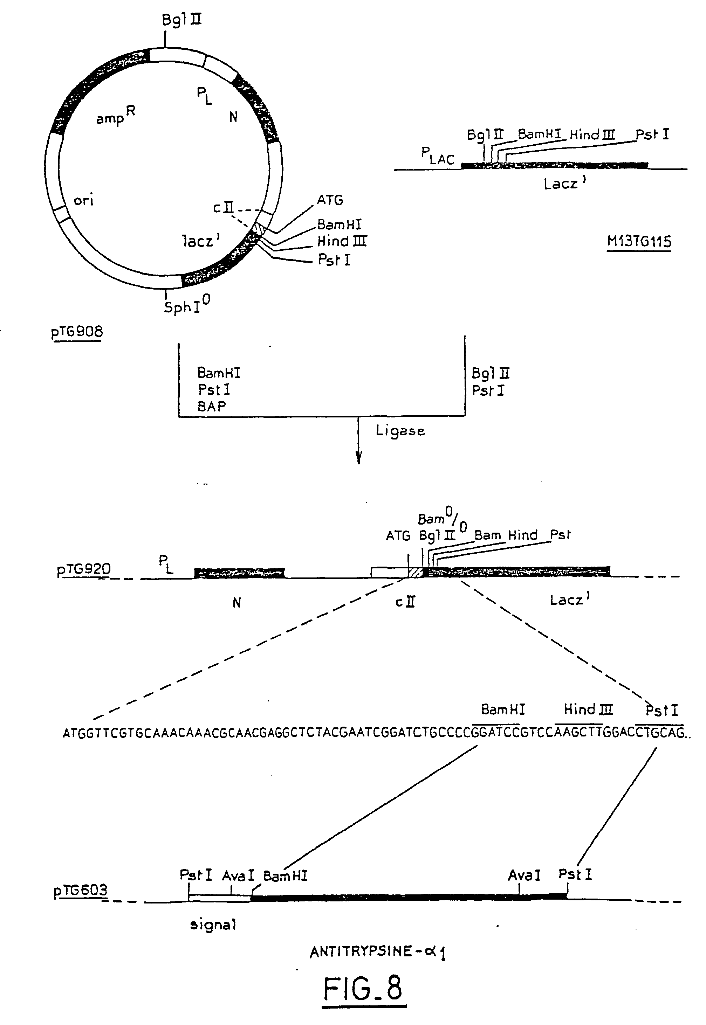

Une région de liaison comportant des sites de restriction uniques est située à la jonction de cII et de la séquence lacZ'. Le plasmide pTG920 est un dérivé de pTG908 préparé dans l'exemple 3, dans lequel le fragment original BamHI/PstI situé à 40 bp en aval de l'ATG du cII dans pTG908, est remplacé par un fragment BglII/PstI de M13tg115, comme cela est décrit dans la figure 8.

Grâce à ce processus, on obtient un site BamHI qui est placé dans la même phase de traduction que le site BamHI du gène de l'antitrypsine-$\alpha_1$.

La figure 8 représente la stratégie de clonage permettant de préparer le plasmide pTG920 à partir du plasmide pTG908 et du phage M13tg115 et indication du fragment à cloner de pTG603. Les traits pleins représentent les séquences codant pour une protéine et dans le cas de pTG603 représentent la séquence codant pour le polypeptide mature de l'antitrypsine-$\alpha_1$. Les régions hachurées représentent les régions codant pour les 13 aminoacides N-terminaux de cII qui seront finalement fusionnés avec l'antitrypsine-$\alpha_1$.

Ainsi, l'insertion d'un fragment BamHI/PstI de pTG603 entre BamHI et le site PstI de pTG920 conduira à l'expression d'un polypeptide fusionné contenant (à partir du $NH_2$ terminal) 13 aminoacides de la protéine cII, 4 aminoacides dérivant de la séquence de l'adaptateur et le polypeptide mature de l'antitrypsine-$\alpha_1$, excepté l'acide glutamique du $NH_2$ terminal.

pTG920, traité par BamHI/PstI et de la phosphatase alcaline, est lié avec pTG603, digéré avec BamHI et PstI. Les cellules TGE900 transformantes portant le fragment l'antitrypsine-$\alpha_1$ sont isolées après étude de restriction enzymatique. L'un de ces clones, pTG922, est choisi pour être étudié plus complètement.

Comme cela a déjà été mentionné, les vecteurs selon l'invention peuvent permettre de préparer, à titre de variante du procédé précédent, la protéine non fusionnée. Pour ce faire, on digère pTG920 avec NdiI et pTG603 avec BamHI comme précédemment, puis on effectue la ligation des fragments obtenus par l'intermédiaire d'un adaptateur oligomère, comme cela est décrit ci-dessous, ou bien on effectue les mêmes opérations sur le vecteur pTG922:

**EP 0 114 777 B1**

```
NdeI                    BamHI
  ↓      cII              ↓
5'-CATATG-------    -------GGATCC 3'
3'-GTATAC-------    -------CCTAGG 5'
  ↑                        ↑
NdeI                    BamHI
```

Adaptateur          TATGGAG            7mer
                    ACCTCCTAG          9mer
                         ↓

```
                         ---
------CA TATGGAG GAT-------
------GT ATACCTC CTA-------
```

Cette jonction reconstitue le codon de départ ATG auquel est fusionné le gène codant pour l'antitrypsine-$\alpha_1$.

Les cellules de la souche *E. coli* TGE900 contenant, soit le plasmide d'expression pTG922 qui contient le gène de l'antitrypsine-$\alpha_1$ humaine, soit le plasmide seul sans insert, pTG920, les protéines sont marquées avec de la méthionine S$_{35}$ pendant 1 heure aux températures indiquées ci-dessus. Les extraits sont préparés comme cela est décrit dans la référence 7 et les aliquots analysés sur un gel de polyacrylamide 10% SDS, suivi par une fluorographie et autoradiographie.

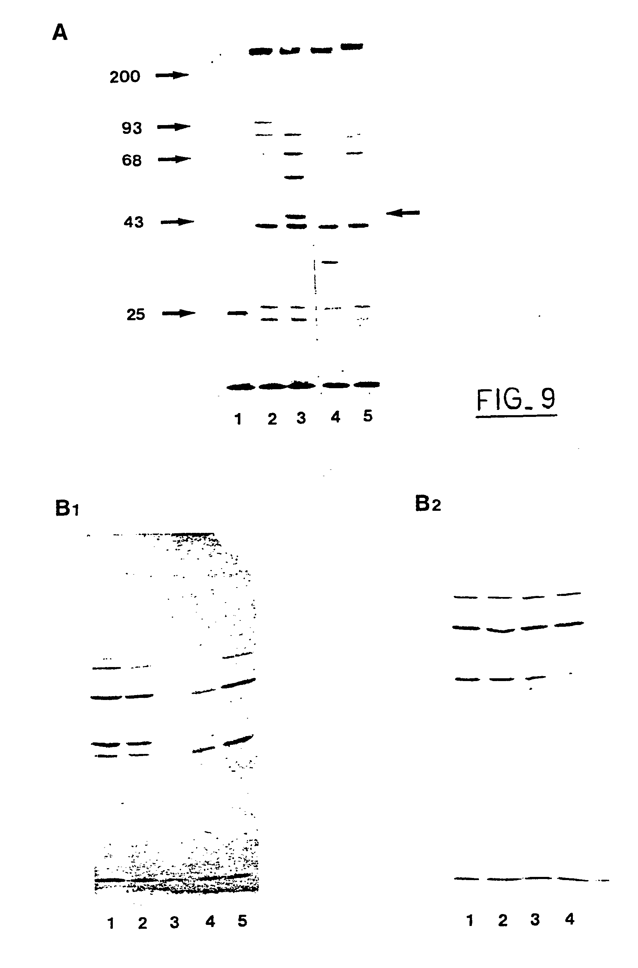

Les résultats sont représentés sur la figure 9A:

Ligne 1: marqueur de poids moléculaire radioactif,

Ligne 2: extrait cellulaire avec pTG922, croissance à 28°C (sans induction),

Ligne 3: comme pour la ligne 2, mais croissance à 42°C (induction),

Ligne 4: extrait de cellules contenant le plasmide pTG920, croissance à 28°C,

Ligne 5: comme pour la ligne 4, mais croissance à 42°C.

Les résultats observés démontrent l'induction d'une bande forte ayant un poids moléculaire d'environ 45 000 daltons (figure 9A). La densitométrie de l'autoradiogramme indique que la quantité d'antitrypsine-$\alpha_1$ produite représente entre 7,5 et 15% des protéines totales de *E. coli* (Il n'a été fait aucun ajustement pour les différences potentielles entre le contenu en méthionine de l'antitrypsine-$\alpha_1$ et celui des protéines de *E. coli* totales).

Les extraits marqués de cellules de *E. coli* portant les plasmides pTG920 et pTG922 sont préparés comme mentionné ci-dessus. Des aliquots sont soumis à une immunoprécipitation avec un antisérum anti-antitrypsine-$\alpha_1$, comme cela est décrit pour la figure 5, et sont analysés sur un gel de polyacrylamide 10% SDS, suivi par une fluorographie. Les résultats sont représentés sur la figure 9B1:

la ligne 1 correspond aux extraits de *E. coli* TGE900 contenant pTG922 (présentant la séquence de l'antitrypsine-$\alpha_1$) marqués à 42°C et immunoprécipités avec l'antisérum de l'antitrypsine-$\alpha_1$, 100 µl d'extraits,

ligne 2, comme pour la ligne 1, mais avec 2 µl d'extraits,

Ligne 3, néant.

Ligne 4, comme pour la ligne 1, avec une croissance cellulaire à une température non permise (28°C), 100 µl d'extraits,

ligne 5, extraits de cellules contenant le plasmide parent pTG920, marqués à la température de l'induction, 42°C, 100 µl d'extraits.

Enfin, sur la figure 9B2 figurent les résultats d'immunocompétition de l'antitrypsine-$\alpha_1$ synthétisée par *E. coli*. Les extraits cellulaires de *E. coli* contenant pTG922 sont marqués à 42°C comme cela a été décrit précédemment. Des aliquots sont utilisés pour les immunoprécipitations suivantes:

ligne 1: immunoprécipitation avec l'antisérum anti-antitrypsine-$\alpha_1$,

ligne 2: comme pour la ligne 1, mais en présence de 10 µg d'antitrypsine-$\alpha_1$ humaine native non marquée,

ligne 3, comme pour la ligne 2, mais 20 µg de compétiteur antitrypsine-$\alpha_1$,

ligne 4, comme pour la ligne 3, mais avec 50 µg de compétiteur.

Ces essais démontrent que le produit d'immunoprécipitation a le même poids moléculaire que celui qui est précipité à partir du produit de traduction exempt de cellule provenant du mARN de foie humain en l'absence de membrane.

18

3) Activité biologique de l'antitrypsine-$\alpha_1$ produite dans *E. coli*

Les extraits bruts de pTG922 sont préparés et des aliquots sont testés pour leur activité anti-élastase de pancréas de porcs.

Pour tester l'activité biologique de l'antitrypsine-$\alpha_1$ préparée par *E. coli* on prépare des extraits bruts de culture comme cela est décrit précédemment en l'absence d'inhibiteur de protéase, sauf un inhibiteur de la trypsine de soja. Les essais à l'élastase sont effectués selon la méthode de Werb (référence 29) modifiée. Les extraits bruts d'échantillons d'antitrypsine-$\alpha_1$ sont préincubés avec 0,25 µg d'élastase pancréatique de porcs pendant 30 minutes à la température ambiante avant d'y ajouter un substrat d'élastine $H^3$ et incubation à 37°C. pendant 15 heures. Les échantillons de 300 µl sont ensuite centrifugés et le pourcentage d'inhibition est déterminé par comptage des surnageants.

Les échantillons sont les suivants:

1) — élastase

2) — élastase + 50 µl d'extraits de pTG920 induits,

3—4) — élastase + 20 µl d'extraits de pTG920 induits + 5 µg et 10 µg d'antitrypsine-$\alpha_1$ humaine (Sigma)

5—6—7) — élastase + 5 µl + 20 µl + 50 µl d'extraits de pTG922 induits,

8) — élastase + 50 µl d'extraits de pTG922 non induits.

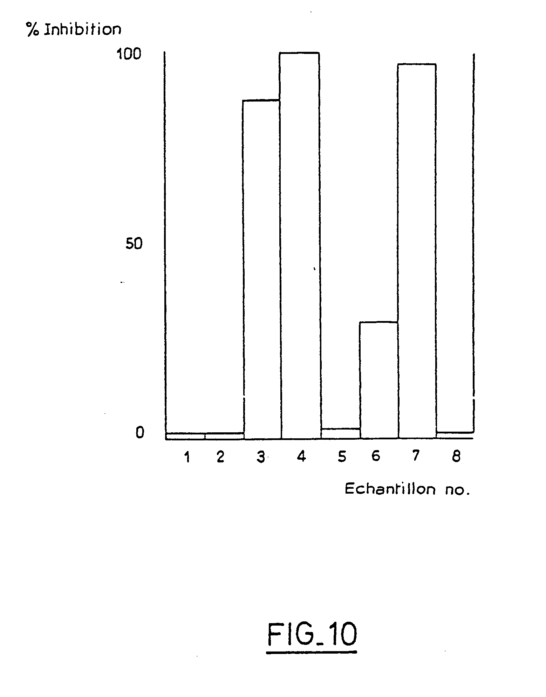

Les résultats montrent (figure 10) qu'une inhibition complète de l'élastase est obtenue seulement avec des extraits induits par pTG922 et non pas dans les extraits non induits de pTG922 ou de pTG920, ce qui indique la présence de l'antitrypsine-$\alpha_1$ active biologiquement dans les extraits induits.

4) Estimation de la quantité d'antitrypsine-$\alpha_1$ produite par les bactéries

A partir des gels de polyacrylamide des extraits induits marqués, on estime qu'environ 15% des protéines cellulaires totales sont constituées par de l'antitrypsine-$\alpha_1$.

En admettant 1 pg de protéines par cellule, ceci donne 0,15 pg d'antitrypsine-$\alpha_1$ par cellule.

Après traitement aux ultrasons et centrifugation, environ 50% de l'antitrypsine-$\alpha_1$ est perdue dans le culot: le pourcentage d'antitrypsine-$\alpha_1$ par rapport au total des protéines cellulaires dans le surnageant est donc de 7,5%.

Une estimation de la quantité d'antitrypsine-$\alpha_1$ produite peut également être faite à partir des mesures de l'activité anti-élastase. Il a été trouvé que 100% d'inhibition pour 0,25 µg d'élastase pancréatique porcine sont obtenus avec 10 µg d'antitrypsine-$\alpha_1$ humaine préparée à partir de sérum (en présence des extraits bactériens de contrôle). Un niveau équivalent d'inhibition est observé avec 50 µl d'extraits bactériens induits contenant de l'antitrypsine-$\alpha_1$, c'est-à-dire:

50 µl d'extraits = 10 µg antitrypsine-$\alpha_1$

<=> 1,5 µl de culture = 10 µg antitrypsine-$\alpha_1$

<=> $1,5 \times 10^8$ cellules = 10 µg antitrypsine-$\alpha_1$

<=> 150 cellules = 10 µg antitrypsine-$\alpha_1$

<=> 1 cellule = ~0,05 pg (i.e. 6% protéines cellulaires totales)

Ceci est en bon accord avec le taux de 7,5% estimé à partir des gels après traitement sonore et centrifugation. Ce calcul indique que l'antitrypsine-$\alpha_1$ produite par des bactéries présente un taux d'activité anti-élastase comparable à l'antitrypsine-$\alpha_1$ préparée à partir de sérum et que l'antitrypsine-$\alpha_1$ est produite avec un taux d'environ 15 mg/l de cultures.

5) Modification post-synthétique de l'antitrypsine-$\alpha_1$

L'antitrypsine-$\alpha_1$ humaine est une glycoprotéine qui contient trois chaînes latérales d'hydrates de carbone. Ces chaînes sont de type N-glucoside contenant de l'acide N-acétyl neuraminique, du galactose, du mannose et de la N-acétyl glycosamine, et sont liées à la protéine par l'intermédiaire d'un radical asparginyl. Les oligosaccharides existent, soit sous forme A (ou à 2 points de branchement), soit sous forme B (un seul point de branchement).

La glycosylation n'est probablement pas nécessaire pour l'activité biologique de la protéine car le produit obtenu à partir des bactéries est actif.

Il peut être nécessaire de glycosyler le produit obtenu par la bactérie de façon à assurer une meilleure stabilité. Ceci est possible *in vitro* car le produit de traduction exempt de cellules, obtenu à partir du mARN de l'antitrypsine-$\alpha_1$ en présence de membrane, voit sa taille augmenter compte tenu de la glycosylation.

Exemple 4
Obtention d'$\alpha$-antitrypsine non fusionnée (fig. 11)

Le plasmide de départ est le plasmide pTG922 décrit précédemment.

Le plasmide pTG922 est soumis à une restriction complète avec les enzymes de restriction *Nde*I (New England Biolabs) et *Bam*HI (Bethesda Research Labs) en utilisant les conditions qui sont indiquées par le fournisseur.

On synthétise par des procédés connus des oligonucléotides adaptateurs complémentaires non phosphorylés ayant la structure suivante:

5'-dTATGGAG-3' et 5'-dGATCCTCCA-3'.

Ces oligonucléotides sont préhybridés puis soumis à la ligation à 4°C avec le plasmide pTG922 qui a été soumis à la restriction enzymatique dans un rapport molaire de 50:1 en utilisant de l'ADN ligase dans des conditions connues.

Le mélange de ligation est utilisé pour transformer des cellules compétentes de la souche TGE900 et les transformants obtenus sont étalés sur un milieu de culture en présence d'ampicilline.

Les colonies sont sélectionnées sur des filtres de nitrocellulose par hybridation avec une sonde marquée à la T4 polynucléotide kinase 5'-dCCTGGGATCCTCCA-3'. Cette sonde complémente entièrement la construction non fusionnée que l'on désire sélectionner mais ne complémente que 7 des nucléotides du plasmide parental pTG922, ceci est insuffisant pour assurer une hybridation.

on obtient ainsi 6 candidats positifs.

Ceux-ci sont mis en culture à 28°C et à 42°C et les extraits préparés sont testés pour leur activité anti-élastase comme cela a été décrit dans le brevet principal.

Les résultats sont rassemblés au tableau ci-joint.

## TABLEAU

| PLASMIDE | TEMPERATURE | % D'INHIBITION D'ELASTASE |
|---|---|---|
| pTG920 | 28°C | 5 |
|  | 42°C | 4,2 |
| pTG922 | 28°C | 93,6 |
|  | 42°C | 98,9 |
| pTG929[1] | 28°C | 4,9 |
|  | 42°C | 60 |
| pTG929[2] | 28°C | 17,7 |
|  | 42°C | 64,4 |

pTG920 est le vecteur seul sans la séquence codant pour l'anti-trypsine-$\alpha_1$,

pTG922

pTG929 (1) et (2) sont différents clones préparés par le procédé de la présente invention.

Les résultats précédents démontrent que les plasmides selon l'invention assurent l'expression d'une antitrypsine-$\alpha_1$ humaine non fusionnée dans *Escherichia coli.*

La diminution du niveau d'expression de l'antitrypsine-$\alpha_1$ humaine dans le plasmide pTG929 par rapport au plasmide pTG922 est probablement due à la disparition d'une partie de la séquence de c11.

### Exemple 5

Le plasmide pTG929 ne produit que des quantités faibles d'α-AT (moins de 0,1% des protéines cellulaires totales). De façon à augmenter le niveau d'expression, on remplace cllrbs par un site rbs synthétique:

```
                                        ┌─traduction
          TCGATAACACAGGAACAGATCTATG
                          ↑
          séquence  Shine/Dalgano
```

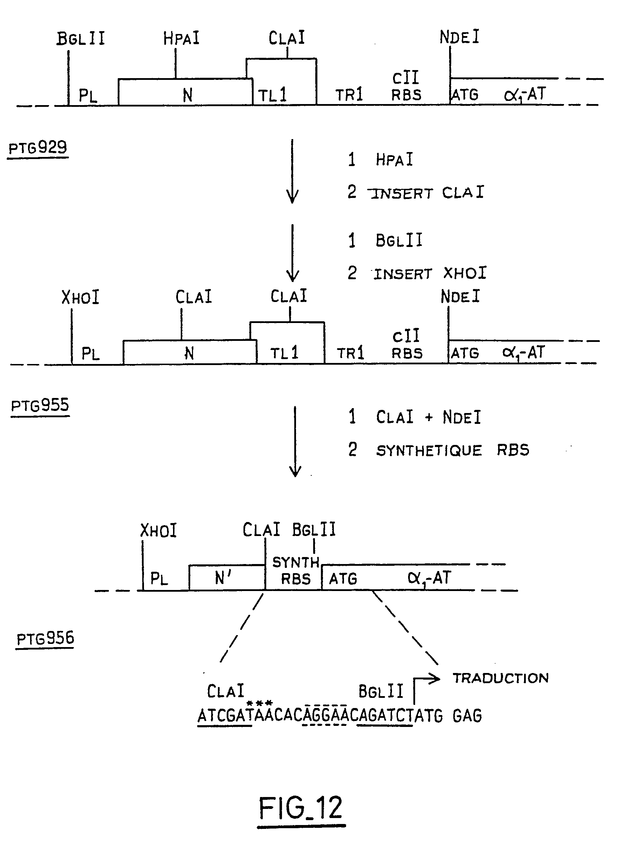

Cet échange est effectué comme cela est représenté à la figure 12. Les sites Hpal et Bglll de pTG929 sont remplacés en utilisant des inserts synthétiques par des sites Clal et Xhol respectivement.

Le rbs synthétique (synth rbs) est alors inséré entre le site NdeI et le nouveau site ClaI créé dans le gène N. Cette manipulation élimine cIIrbs et conduit à un gène N tronqué immédiatement suivi de synth rbs. Un codon de terminaison de la traduction (TAA) dans synth rbs bloque la traduction de N. Le plasmide obtenu, pTG956, produit $\alpha_1$-AT au niveau de 0,1% des protéines cellulaires totales comme cela est mis en évidence par l'essai anti-élastase *in vitro*.

## Exemple 6

Comme cela a été indiqué dans la description, on remplace dans le plasmide pTG956 la séquence originale de l'$\alpha$-AT par une séquence mutée:

séquence originale :

protéine : met glu asp pro glu gly asp ala

ADN      : ATG GAG GAT CCC CAG GGA GAT GCT

séquence mutée :

protéine : met glu asp pro glu gly asp ala

ADN      : ATG GAA GAT CCT CAA GGC GAT GCT

                  ✱           ✱   ✱   ✱

Chaque changement (noté par un asterisque) est effectué en position 3 des codons et ne modifie pas l'aminoacide codé. Le gène de l'$\alpha$-AT a été modifié en utilisant la technique de mutagénèse dirigée sur sites en utilisant un oligonucléotide synthétique qui définit les changements de base particuliers (réf. 30). Les changements de séquence choisis proviennent d'une étude statistique qui démontre une préférence pour certaines bases dans plusieurs positions au voisinage du début des gènes de E. coli (réf. 31 et 32). Ces changements déstabiliseront également les régions correspondant à des structures secondaires possibles qui peuvent apparaître dans la séquence de mARN de l'$\alpha$-AT. L'oligonucléotide pour la mutagénèse est:

5'-AGCATCGCCTTGAGGATCTTCCAT-3'

Cette séquence contient 4 différences par rapport à la séquence originale et conduit aux modifications indiquées précédemment dans le gène de l'$\alpha$-AT.

Le plasmide obtenu, pTG983, est identique à pTG956 à l'exception des changements mentionnés précédemment.

## Exemple 7

Resultats observes par immunoprecipitation et par degradation de l'elastase de leucocytes humains Deux séries d'essais ont été réalisées:

immunoprécipitation de cultures de souches marquées à la [$^{-35}$S]-méthionine avec un antisérum anti $\alpha_1$AT,

et activité anti-élastase (issue de leucocytes humains).

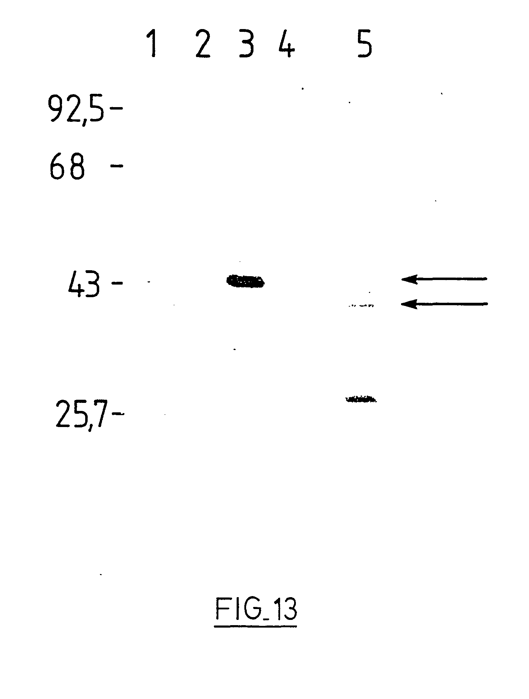

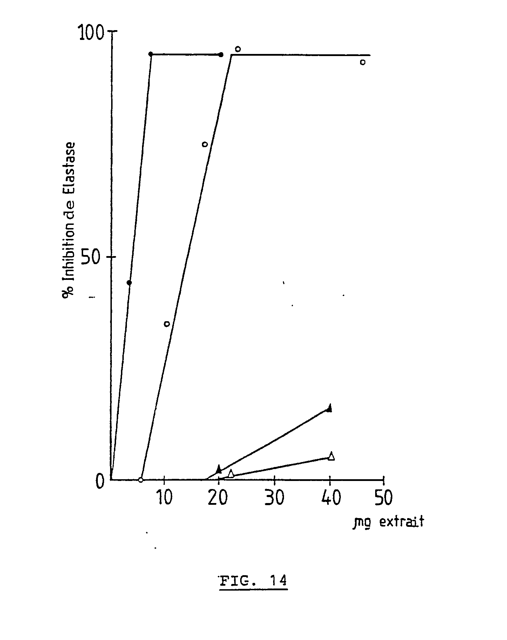

Les résultats de ces essais sont illustrés aux figures 13 et 14.

Sur la figure 13 apparaît les résultats de 5 essais d'immunoprécipitation:

cylindre 1: culture-témoin (poids moléculaires en kilodaltons),

cylindre 2: culture de pTG920 (vecteur plasmidique sans insert) induit,

cylindre 3: culture de pTG922 ($\alpha_1$AT fusionnée) induit,

Cylindre 4: culture de pTG922 ($\alpha_1$AT fusionnée) non induit,

cylindre 5: culture de pTG983 ($\alpha_1$AT non-fusionnée) induit.

Les flèches indiquant les zones de précipitation correspondant à l'$\alpha_1$AT fusionnée (bande supérieure) et l'$\alpha_1$AT non-fusionnée (bande inférieure). La bande inférieure apparaissant sur le cylindre 5 résulte probablement d'une initiation de traduction interne à la séquence d'$\alpha_1$AT.

Cette première série de tests permet de mettre en évidence que l'immunoprécipitation avec un antisérum anti-$\alpha_1$AT de protéines marquées provenant de cultures de souches contenant pTG983 (souche-hôte TGE 900), permet d'obtenir une protéine de poids moléculaire de 42 kilodaltons, ce qui correspond à la taille de l'$\alpha_1$AT non-fusionnée (cf. cylindre 5), par comparaison avec la protéine fusionnée de 44 kilodaltons obtenue à partir de cultures de souches de pTG922 (cylindre 3).

Sur la figure 14 sont indiqués, de façon schématique, les résultats des essais d'activité anti-élastase. Ceux-ci ont été réalisés à partir d'une élastase de leucocytes humains (50 ng) (Elastin Products CO. Inc.) et de méthoxy-succinyl-ala-ala-pro-val-nitroanilide (CALBIOCHEM) comme substrat. Le clivage de l'élastase libère la nitroanilide qui est récupérée à 410 nm.

## EP 0 114 777 B1

Sur le graphique de la figure 14, la légende des différents tracés est la suivante:

— ● — extrait de pTG922 induit
— ○ — extrait de pTG983 induit
— ▲ extrait de pTG983 non induit
— △ extrait de pTG920 témoin induit

Ces tests anti-élastase effectués in vivo montrent que pTG983 permet l'expression de taux importants d'$\alpha_1$AT. La figure 14 montre qu'une inhibition de 50% d'élastase (50 ng) est obtenue avec environ 4 µg d'un extrait de pTG922 et avec environ 14 µg d'un extrait de pTG983. Ceci correspond respectivement à environ 3% et environ 1% de protéine cellulaire totale produite. 50 ng d'élastase sont inhibés à 50% par 100 à 120 ng d'$\alpha_1$AT humaine naturelle (Sigma). La différence entre les pourcentages d'$\alpha_1$AT obtenus avec les uns et les autres des essais effectués avec pTG922, résulte de la variation de la quantité d'$\alpha_1$AT, perdue, cette perte correspondant à une précipitation intracellulaire. Aucunes de ces précipitations intracellulaires n'ont pu être mises en évidence avec les cultures de pTG983.

Références
1. Gadek J.E. et Crystal R.G. (1982) "$\alpha_1$-Antitrypsin in the Metabolic Basis of Inherited Disease, 5th edition, eds Stanbury Wyngaarden, Fredrickson, Goldstein et Brown. Mc Graw Hill.
2. Fagerhol M.K. et Cox D.W. (1981), The Pi Polymorphism Genetic, Biochemical and Clinical Aspects of Human $\alpha_1$-Antitrypsin in Advances in Human Genetics, 11, 1—62.
3. Küppers F. (1973), Am. J. Hum. Genet. 25, 667—686.
4. Gadek J.E., Fells G.A. et Crystal R.G. (1979), Cigarette smoking induces functional antiprotease deficiency in the lower respiratory tract of humans; Science 206, 1315—1316.
5. Carp H. et Janoff A. (1977), Possible mechanisms of emphysema in smokers; Cigarette smoke condensate suppresses protease inhibition in vitro; Am. Rev. Resp. Dis. 116, 65.
6. Gadek J.E., Klein H.G., Holland P.V. et Crystal R.G. (1981), Replacement Therapy of alpha 1-Antitrypsin Deficiency. Reversal of Protease-Antiprotease Imbalance Within the Alucolar Structures of Pi Z Subjects. J. Clin. Invest. 68, 1158—1165.
7. Groopman J.E. et Gottlieb M.S. (1982), Karposi's Sarcoma; an oncological looking glass. Nature 299, 103—104.
8. Remant E., Staussens P. et Fiers W., Gene 15, 81—93 (1981).
9. Yoakum G.H., Young A.T., Mattes W.B. et Grossman L., Proc. Natl. Acad. Sci. USA, 79, 1766—1770 (1982).
10. Herskowitz I. et Signer E., J. Mol. Biol. 47, 545—556 (1970).
11. Grayhack E.U. et Roberts J.W., Cell, 30, 637—648 (1982).
12. Ish-Horowitz D. et Burke J.F., Nucl. Acids Res. 9, 2989—2998 (1981).
13. Panayotatos N. et Trong K., Nucl. Acids Res. 9, 5679—5688 (1981).
14. Hoopes B.C. et McClure R.R., Nucl. Acids Res. 9, 5493—5504 (1981).
15. Dagert M. et Ehrlich S.D., Gene, 23—28 (1979).
16. Vieira J. et Messing J., Gene 19, 259—268.
17. Shimatake H. et Rosenberg M., Nature, 292, 128—132, (1981).
18. Oppenheimer A.B., Gottesman S. et Gottesman M., J. Mol. Biol., 158, 327—346 (1982).
19. Daniels D.L. et Blattner F.R., Virology, 117, 81—92, (1982).
20. Tolstoshev P., Berg R.A., Rennard S.I., Bradley K.H., Trapnell B.C. et Crystal R.G. (1981), Procollagen Production and procollagen messenger RNA levels and activité in human lung fibroblasts during periods of rapide and stationary growth, J. Biol. Chem. 256, 3135—3140.
21. Blobel G. et Dobberstein B. (1975), J. Cell. Biol. 67, 852—862.
22. Lathe R., Hirth P., De Wilde M., Hartford N. et Lecocq J.P. (1980), Nature 284, 473—474.
23. Dagert M. et Ehrlich S.D. (1979) Gene 6, 23—28.
24. Kurachi K., Chandra T., Degen S.J.F., White T.T., Marchioro T.L., Woo S.L.C. et Davie E.W. (1981), Proc. Natl. Acad. Sci. USA, 78, 6826—6830.
25 Kohli V., Balland A. et Lecocq J.P. (1982) Brevet n° 82 12100.
26. Suggs S.V., Wallace R.B., Hirose T., Kawashima E.H. et Itakura K. (1981) Proc. Natl. Acad. Sci. USA, 78, 6613—6617.
27. Birnboim H.C. et Doly J. (1979) Nucleic Acids Res. 7, 1513—1523.
28. Leicht M., Long G.L., Chandra T., Kurachi K., Kidd V.J., Mace M. Jr., Davie E.W. et Woo S.L. (1982) Nature 297, 655—659.
29. Werb Z. (1981) Biochem J. 193, 589—605.
30. Zoller, M.J. and Smith, M. (1983) In Methodo of Enzymology, Vol. 100 pp 468—500.
31. Scherer, G.F.E., Walkinshaw, M.D., Arnott, S. and Motté D.J. (1980) Nucl. Acids. Res. 8, 3895—3907.
32. Gold, L., Pribnow, D., Schneider, T., Shinedling, S., Singer, B.S. and Stormo, G. (1981) Ann. Rev. Microbiol. 35, 365—403.

# EP 0 114 777 B1

**Revendications**

1. Bactérie transformée exprimant l'antitrypsine alpha-₁ humaine, caractérisée en ce que ladite bactérie est transformée par un vecteur capable d'exprimer dans cette bactérie l'antitrypsine alpha-₁ humaine, ledit vecteur comportant une séquence codant pour l'antitrypsine alpha-₁ humaine, sous le contrôle d'un promoteur et une région d'initiation de la traduction ayant la structure suivante:

```
                                        ┌traduction
                                        └
        TCGATAACACAGGAACAGATCTATG
                    ------
```

**séquence Shine/Dalgano**

ainsi qu'une origine de réplication efficace dans ladite bactérie.

2. Bactérie selon la revendication 1, caractérisée en ce que le vecteur comporte au voisinage du codon de départ de la traduction du gène codant pour l'antitrypsine alpha-₁ humaine la structure suivante:

```
        ATG GAA GAT CCT CAA GGCGATGCT

        TAC CTT CTA GGA GTT CCGCTACGA
```

3. Bactérie transformée exprimant l'antitrypsine alpha-₁ humaine, caractérisée en ce que ladite bactérie est transformée par un vecteur capable d'exprimer dans cette bactérie l'antitrypsine alpha-₁ humaine, ledit vecteur comportant une séquence codant pour l'antitrypsine alpha-₁ humaine, sous le contrôle d'un promoteur et une région d'initiation de la traduction, dans laquelle la structure au voisinage du codon ATG est la suivante:

```
        TCGATAACACAGGAACAGATCTATG GAA GAT CCT CAA GGC GAT GCT
```

4. Bactérie selon l'une des revendications 1 à 3, caractérisée en ce que le promoteur utilisé est le promoteur $P_L$.

5. Bactérie selon l'une des revendications 1 à 3, caractérisée en ce que le vecteur comporte, en outre, une fonction d'antiterminaison de transcription.

6. Bactérie selon la revendication 5, caractérisée en ce que la fonction d'antiterminaison de transcription est le gène lambda N pour le promoteur $P_L$ ou $P_R$ ou le gène lambda Q pour le promoteur $P'_R$.

7. Bactérie selon l'une des revendications 1 à 6, caractérisé en ce que le vecteur d'expression comporte l'origine de réplication de pBR322.

8. Bactérie selon l'une des revendications 1 à 7, caractérisé en ce que le vecteur d'expression comporte un gène codant pour la résistance à un antibiotique.

9. Bactérie selon la revendication 8, caractérisé en ce que le vecteur d'expression comporte un gène de résistance à l'ampicilline.

10. Bactérie selon l'une des revendications 1 à 9, caractérisée en ce que le promoteur est contrôlée par un répresseur.

11. Bactérie selon la revendication 10, caractérisée en ce que le répresseur est thermosensible.

12. Bactérie selon l'une des revendications 1 à 11, caractérisée en ce qu'il s'agit d'une souche de *E. coli*.

13. Procédé de préparation d'antitrypsine alpha-₁ humaine d'origine bactérienne, caractérisée en ce qu'on cultive sur un milieu de culture une bactérie transformée selon l'une des revendications 1 à 12.

**Patentansprüche**

1. Transformiertes Bakterium, das Human-Alpha₁-Antitrypsin exprimieren kann, dadurch gekennzeichnet, daß das Bakterium transformiert ist durch einen Vektor, der in dieses Bakterium Human-Alpha₁-Antitrypsin exprimieren kann und der aufweist eine Sequenz, die kodiert für Human-Alpha₁-Antitrypsin unter der Kontrolle eines Promoters und einen Translationsinitiierungsbereich mit der folgenden Struktur

```
                                        ┌Translation
                                        └
        TCGATAACACAGGAACAGATCTATG

                    -----
```

**Shine /Dalgarno-Sequenz**

sowie einen wirksamen Replikationsursprung in dem Bakterium.

23

2. Bakterium nach Anspruch 1, dadurch gekennzeichnet, daß der Vektor in der Nähe des Ausgangs-Codons für die Translation des Gens, das für Human-Alpha$_1$-Antitrypsin kodiert, die folgende Struktur aufweist:

ATG GAA GAT CCT CAA GGCGATGCT

TAC CTT CTA GGA GTT CCGCTACGA

3. Transformiertes Bakterium, das Human-Alpha$_1$-Antitrypsin exprimiert, dadurch gekennzeichnet, daß das Bakterium durch einen Vektor transformiert ist, der in dieses Bakterium Human-Alpha$_1$-Antitrypsin exprimieren kann und der aufweist eine Sequenz, die für Human-Alpha$_1$-Antitrypsin kodiert unter der Kontrolle eines Promoters und einen Translationsinitiierungsbereich aufweist, in dem die Struktur in der Nähe des ATG-Codons die folgende ist:

TCGATAACACAGGAACAGATCTATG GAA GAT CCT CAA GGC GAT GCT

4. Bakterium nach einem der Ansprüche 1 bis 3, dadurch gekennzeichnet, daß es sich bei dem verwendeten Promotor um den Promotor P$_L$ handelt.

5. Bakterium nach einem der Ansprüche 1 bis 3, dadurch gekennzeichnet, daß der Vektor außerdem eine Transkriptions-Antiterminationsfunktion aufweist.

6. Bakterium nach Anspruch 5, dadurch gekennzeichnet, daß es sich bei der Transkriptions-Antiterminationsfunktion um das Gen Lambda N für den Promotor P$_L$ oder P$_R$ oder um das Gen Lambda Q für den Promotor P'$_R$ handelt.

7. Bakterium nach einem der Ansprüche 1 bis 6, dadurch gekennzeichnet, daß der Expressionsvektor den Replikationsursprung von pBR322 aufweist.

8. Bakterium nach einem der Ansprüche 1 bis 7, dadurch gekennzeichnet, daß der Expressionsvektor ein Gen aufweist, das für die Resistenz gegenüber einem Antibiotikum kodiert.

9. Bakterium nach Anspruch 8, dadurch gekennzeichnet, daß der Expressionsvektor ein Gen für die Resistenz gegenüber Ampicillin aufweist.

10. Bakterium nach einem der Ansprüche 1 bis 9, dadurch gekennzeichnet, daß der Promotor durch einen Repressor kontrolliert wird.

11. Bakterium nach Anspruch 10, dadurch gekennzeichnet, daß der Repressor wärmeempfindlich ist.

12. Bakterium nach einem der Ansprüche 1 bis 11, dadurch gekennzeichnet, daß es sich um einen Stamm von E. Coli handelt.

13. Verfahren zur Herstellung von Human-Alpha$_1$-Antitrypsin, das aus Bakterien stammt, dadurch gekennzeichnet, daß man auf einem Kulturmedium ein nach einem der Ansprüche 1 bis 12 transformiertes Bakterium kultiviert.

## Claims

1. A transformed bacterium for expressing human alpha-, antitrypsin, characterised in that the bacterium is transformed by a vector capable of expressing human alpha-, antitrypsin in the bacterium, the vector comprising a sequence which codes for human alpha-$_1$, antitrypsin under the control of a promoter, and a translation initiating region having the following structure:

┌►Translation

TCGATAACACAGGAACAGATCTATG

-----

Shine /Dalgarno-Sequenz

and an origin of replication which is operative in the bacterium.

2. A bacterium according to claim 1, characterised in that the vector, near the translation-initiating codon of the gene which codes for human alpha-$_1$, antitrypsin, comprises the following structure:

ATG GAA GAT CCT CAA GGCGATGCT

TAC CTT CTA GGA GTT CCGCTACGA.

3. A transformed bacterium which expresses human alpha-$_1$, antitrypsin, characterised in that the bacterium is transformed by a vector capable of expressing human alpha-$_1$, antitrypsin in the bacterium, the vector comprising a sequence which codes for human alpha-$_1$, antitrypsin under the control of a promoter, and a translation-initiating region in which the structure near the ATG codon is as follows:

TCGATAACACAGGAACAGATCTATG GAA GAT CCT CAA GGC GAT GCT.

4. A bacterium according to any of claims 1 to 3, characterised in that the promoter used is $P_L$.

5. A bacterium according to any of claims 1 to 3, characterised in that the vector also comprises a transcription anti-termination group.

6. A bacterium according to claim 5, characterised in that the transcription anti-termination group is the lambda N gene for the promoter $P_L$ or $P_R$ or the lambda Q gene for the promoter $P'_R$.

7. A bacterium according to any of claims 1 to 6, characterised in that the expressing vector comprises the origin of replication of pBR322.

8. A bacterium according to any of claims 1 to 7, characterised in that the expressing vector comprises a gene which codes for resistance to an antibiotic.

9. A bacterium according to claim 8, characterised in that the expressing vector comprises a gene for resistance to ampicillin.

10. A bacterium according to any of claims 1 to 9, characterised in that the promoter is controlled by a repressor.

11. A bacterium according to claim 10, characterised in that the repressor is heat-sensitive.

12. A bacterium according to any of claims 1 to 11, characterised in that it is a strain of E. coli.

13. A method of preparing human alpha-$_1$, antitrypsin of bacterial origin, characterised in that a bacterium transformed in accordance with any of claims 1 to 12 is cultivated on a culture medium.

FIG_1

FIG. 2

FIG_3

FIG_4

EP 0 114 777 B1

# FIG_5

93 →
68 →
43 →
25 →

1   2   3 4

5

```
CCC CTG AAG CTC TCC AAG GCC GTG CAT AAG GCT GTG CTG ACC ATC GAC GAG AAA GGG ACT GAA GCT GCT GGG GCC ATG TTT TTA GAG GCC
Pro Leu Lys Leu Ser Lys Ala Val His Lys Ala Val Leu Thr Ile Asp Glu Lys Gly Thr Glu Ala Ala Gly Ala Met Phe Leu Glu Ala

ATA CCC ATG TCT ATC CCC CCC GAG GTC AAG TTC AAC AAA CCC TTT GTC TTC TTA ATG ATT GAA CAA AAT ACC AAG TCT CCC CTC TTC ATG
Ile Pro Met Ser Ile Pro Pro Glu Val Lys Phe Asn Lys Pro Phe Val Phe Leu Met Ile Glu Gln Asn Thr Lys Ser Pro Leu Phe Met

GGA AAA GTG GTG AAT CCC ACC CAA AAA TAA CTG CCT CTC GCT CCT CAA CCC CTC CCC TCC ATC CCT GGC CCC CTC CCT GGA TGA CAT TAA
Gly Lys Val Val Asn Pro Thr Gln Lys ***
```

## FIG_6

```
5' GGCCATGTTTTTAGAGGCCATACCCATGTCTATCCCCCCCGAGGTCAAGTTCAACAAACCCTTTGTCTTCTTAATGATTGAA 3'
   *****  ******  *    ****  ****************************************  **  ***********************************
   GGCCAXXTTTTTAXAXXCCATTCCCATGTCTATCCCCCCCGAGGTCAAGTTXAAXAAACCCTTTGTCTTCTTAATGATTGAA
```

## FIG_7

EP 0 114 777 B1

FIG. 8

ANTITRYPSINE-α1

7

FIG. 9

% Inhibition

FIG_10

FIG_11

FIG_12

FIG. 13

FIG. 14